(19) Europäisches Patentamt
European Patent Office
Office européen des brevets

(11) Veröffentlichungsnummer: **0 564 499 B1**

(12) # EUROPÄISCHE PATENTSCHRIFT

(45) Veröffentlichungstag der Patentschrift: **12.04.95**

(51) Int. Cl.6: **C07C 311/00**, C07C 311/15, C07C 323/67, C07D 295/088, C07D 295/135, C07D 239/28, C07D 213/60, A61K 31/18, A61K 31/33

(21) Anmeldenummer: **92901147.6**

(22) Anmeldetag: **19.12.91**

(86) Internationale Anmeldenummer: **PCT/EP91/02456**

(87) Internationale Veröffentlichungsnummer: **WO 92/11234 (09.07.92 92/17)**

(54) **NEUE AMINE, VERFAHREN ZU IHRER HERSTELLUNG, SOWIE DIESE VERBINDUNGEN ENTHALTENDE ARZNEIMITTEL.**

(30) Priorität: **24.12.90 DE 4041780**

(43) Veröffentlichungstag der Anmeldung: **13.10.93 Patentblatt 93/41**

(45) Bekanntmachung des Hinweises auf die Patenterteilung: **12.04.95 Patentblatt 95/15**

(84) Benannte Vertragsstaaten: **AT BE CH DE DK ES FR GB GR IT LI LU NL SE**

(56) Entgegenhaltungen:
**EP-A- 0 325 245**
**EP-A- 0 356 989**
**US-A- 4 003 929**
**US-A- 4 122 255**
**US-A- 4 127 606**
**US-A- 4 151 354**

(73) Patentinhaber: **BOEHRINGER MANNHEIM GMBH**

**D-68298 Mannheim (DE)**

(72) Erfinder: **WITTE, Ernst-Christian**
**Beethovenstr. 2**
**D-6800 Mannheim 1 (DE)**
Erfinder: **BECKH, Hansjörg**
**Wolfstr. 7**
**D-6842 Bürstadt (DE)**
Erfinder: **STEGMEIER, Karlheinz**
**Kirchbergstr. 17**
**D-6148 Heppenheim (DE)**
Erfinder: **DOERGE, Liesel**
**Schwalbenstr. 26**
**D-6840 Lampertheim (DE)**

EP 0 564 499 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**CHEMICAL ABSTRACTS, Band 108, Nr. 26, 27. Juni 1988, Columbus, Ohio, USA; Y. DEGUCHI et al, "Manufacture of glycidyl aromatic sulfonamides for chemically resistant epoxy resins", Seite 7, Zusammenfassung Nr. 222242t; & JP-A-62 246 561**

(74) Vertreter: **Weber, Manfred, Dr. et al**
**c/o Boehringer Mannheim GmbH,**
**Patentabteilung,**
**Sandhoferstrasse 116**
**D-68298 Mannheim (DE)**

## Beschreibung

Die vorliegende Erfindung betrifft neue Amine der allgemeinen Formel I

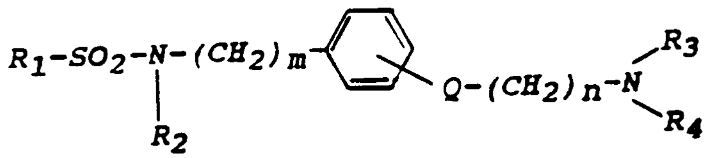 (I),

in welcher

- $R_1$ eine Aryl-, Aralkyl oder eine Aralkenylgruppe, deren Arylrest jeweils ein- oder mehrfach durch Halogen, Cyan, Alkyl, Trifluormethyl, Alkoxy, Alkylthio, Trifluoromethoxy, Hydroxy oder Carboxy substituiert sein kann,
- m eine ganze Zahl 2,
- n eine ganze Zahl von 2 bis 5,
- $R_2$ Wasserstoff,
- Q eine Bindung oder ein Sauerstoffatom,
- $R_3$ Wasserstoff oder eine $C_1$-$C_4$-Alkylgruppe, welche ggf. endständig durch Carboxyl oder durch eine Hydroxygruppe substituiert ist und
- $R_4$ Wasserstoff, eine niedere Alkylgruppe mit 1-4 C-Atomen, welche ggf. endständig durch Carboxyl oder Hydroxyl substituiert ist, eine ggf. substituierte Phenyl-, Pyridinyl-, Pyrimidinyl-, Cycloalkyl- oder Acyl-Gruppe
  oder eine Gruppe

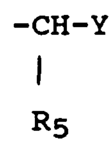

in welcher $R_5$ eine geradkettige oder verzweigte Alkylkette mit 1-4 C-Atomen darstellt, welche ggf. endständig durch Carboxyl, Alkoxycarbonyl, Aminocarbonyl, Hydroxy, Mercapto, Alkylthio oder Imidazolyl substituiert ist und

- Y eine Carboxy, eine Alkoxycarbonyl, Aminocarbonyl- oder Cyano, Formyl, Hydroxymethyl, Aminomethyl oder eine Orthoestergruppe darstellt, bedeuten

wobei $R_3$ und $R_4$ auch Bestandteil eines 5- oder 6-gliedrigen gesättigten oder ungesättigten, ggf. substituierten Heterozyclus mit 1-4 Heteroatomen sein kann, der mit weiteren Ringverbindungen über eine oder mehrere Bindungen annelliert sein kann.

Gegenstand der Erfindung sind also Sulfonamidgruppen enthaltende Phenylalkylamine und Phenoxyalkylamine.

Die Erfindung umfaßt Verbindungen der allgemeinen Formel I, in denen der Sulfonamidoalkyl-Rest sowohl in ortho- als auch in meta- oder para-Stellung zum Rest $-Q-(CH_2)_n-NR_3R_4$ steht. Besonders bevorzugt sind die meta- und die para-Stellung.

Enthalten die Verbindungen der allgemeinen Formel I asymmetrische Kohlenstoffatome, so sind sowohl deren reine optische Isomere (Enantiomere) als auch deren Gemische/Racemate im Anspruch enthalten.

Für den Fall, daß die Verbindungen I Carboxylgruppen enthalten, werden auch deren physiologisch unbedenklichen Salze, Ester und Amide beansprucht.

Aus zwei Veröffentlichungen sind Disulfonamide des Phenylalkylamin-Typs bekannt: D.B. Baird et al. Soc. Perkin Trans. 1, Nr.8 (1973) und J.H. Wood and R.E. Gibson, Am. Soc. 71 (1949), 393. In keiner der beiden angeführten Arbeiten ist eine pharmakologische Wirkung beschrieben worden.

Substituierte Phenylmethylsulfonamide mit antiviraler Wirkung sind aus dem US Patent 4,003,929 und mit entzündungshemmender Wirkung aus den US Patenten 4,122,255, 4,127,606 und 4,151,354 bekannt.

Gegenstand bisheriger Patente/Veröffentlichungen waren außerdem Sulfonamidgruppen enthaltende Phenoxycarbonsäuren sowie deren Ester und Amide, Phenylalkylcarbonsäuren mit Aminosäuren als Aminkomponente, sowie die in der EP-Anmeldung 0 356 989 beanspruchten, Sulfonamidgruppen enthaltenden Tetrazolverbindungen. Alle diese Verbindungen enthalten im Prinzip eine Säurefunktion. Es war daher überraschend, daß die saure Funktion durch eine basische Aminfunktion ersetzt werden kann, ohne daß ein Verlust der beispielsweise in der obengenannten EP-Anmeldung beschriebenen pharmakologischen Aktivität der dort beanspruchten Verbindungen beobachtet wird. Diese Aktivität schließt eine antagonistische Wirkung gegenüber Thromboxan $A_2$ sowie gegen Prostaglandin-Endoperoxide, die Hemmung der Aggregation von Blutplättchen, sowie die Verhinderung der Konstriktion der glatten Muskulatur sowie der Bronchokonstriktion ein.

Die neuen Verbindungen der allgemeinen Formel I zeigen die erwähnten Wirkungen der in der oben genannten EP-Anmeldung beschriebenen Verbindungen und hierbei insbesondere eine ausgezeichnete antagonistische Wirkung gegenüber Thromboxan $A_2$ sowie gegen Prostaglandin-Endoperoxide und verhindern außerdem die Kontraktion von Mesangiumzellen und ähnlichen Zellen mit kontraktilen Eigenschaften.

Diese Wirkung macht sie zu wertvollen Heilmitteln zur Behandlung von cardiovasculären Erkrankungen, wie dem akuten Herz- und Hirninfarkt, cerebraler und coronarer Ischämie, Migräne, peripherer arterieller Verschlußkrankheit sowie venöser und arterieller Thrombosen. Weiterhin kann ihre frühzeitige Anwendung das Auftreten von Organschäden bei Schockpatienten günstig beeinflussen. Sie sind weiter geeignet zur Verhinderung des Thrombozyten- und Leukozytenabfalls bei Eingriffen mit extracorporalem Kreislauf und bei der Hämodialyse. Ihr Zusatz zu Thrombozytenkonzentraten stabilisiert die Blutplättchen und steigert somit die Lagerfähigkeit der Konserven.

Da Thromboxan beim Asthma bronchiale ein Mediator der entzündlichen Reaktion ist, kann durch die Anwendung dieser Thromboxan-Rezeptor-Blocker vor allem die für das chronische Asthma charakteristische Hyperreaktivität abgeschwächt oder sogar beseitigt werden.

Die neuen Thromboxan-Rezeptor-Blocker sind weiter protektiv wirksam bei Gastritis und Ulcusneigung und somit zur Rezidivprophylaxe derselben einsetzbar. In einem Modell der experimentellen akuten Pankreatitis konnte der Verlauf durch den Einsatz eines Thromboxan-Antagonisten gebessert werden. Es ist somit zu erwarten, daß zumindest bestimmte Formen der akuten Pankreatitis beim Menschen in ihrer Prognose durch den Einsatz dieser Thromboxan-Antagonisten verbessert werden können.

Zusätzlich vermögen diese neuen Verbindungen den Acetateinbau in Cholesterin zu hemmen und eignen sich daher auch zur Behandlung von Fettstoffwechselerkrankungen, die mit gesteigerter Cholesterinsynthese einhergehen. Besonders zu betonen ist ihr ausgeprägter antiatherogener Effekt bei erhöhten Serumcholesterinwerten, der sich in einer Reduktion der Plaquebildung besonders in den Coronararterien und in der Aorta zeigt.

Da der Diabetes mit einer gesteigerten Thromboxanbildung einhergeht, lassen sich durch die chronische Anwendung dieser Thromboxan-Antagonisten die typischen Spätschäden an Gefäßen der Niere und am Auge in ihrer Entwicklung verzögern oder sogar verhindern.

Auch bei einer Reihe von immunologisch oder nicht-immunologisch bedingten Nierenerkrankungen, wie z.B. Glomerulonephritis, akutem Nierenversagen, Transplantatabstoß und durch nephrotoxische Substanzen bedingter Nierenschädigung, wurde eine vermehrte Ausscheidung von Thromboxan B2 im Urin beobachtet. Bei diesen Erkrankungen ist eine Intervention mit den neuen Thromboxan-Antagonisten im Hinblick auf die Erhaltung der Nierenfunktion erfolgversprechend.

Da bei Tumorzellen eine vermehrte Thromboxansynthese nachgewiesen wurde und gleichzeitig die Proliferation dieser Zellen durch die Gabe von Thromboxan-Antagonisten gehemmt werden konnte, stellen diese eine wirkungsvolle adjuvante Therapie dar.

Bei der pathologischen Schwangerschaft wird eine Störung des Gleichgewichts der Prostaglandine als ursächlich angesehen. Daher kann durch Blockade der Thromboxan- und PGF2alpha-Rezeptoren insbesondere die vorzeitige Wehentätigkeit unterbrochen werden und bei Schwangerschaftsgestose bzw. Eklampsie ein günstigerer Verlauf erzielt werden. Daneben lassen sich hierdurch auch die prostaglandinbedingten Symptome der Dysmenorrhoe und des prämenstruellen Syndroms therapieren.

Als Arylrest $R_1$, allein oder in Verbindung mit einer Alkyl- oder Alkenylkette, sind in allen Fällen aromatische Kohlenwasserstoffe mit 6-14 C-Atomen, insbesondere der Phenyl-, der Biphenylyl-, der Naphthyl- und der Fluorenylrest, zu verstehen. Diese Arylreste können in allen möglichen Positionen ein-, zwei- oder dreifach substituiert sein, wobei als Substituenten Halogen, $C_1$-$C_6$ Alkyl, $C_1$-$C_6$ Alkoxy, $C_1$-$C_6$ Alkylthio, Trifluormethoxy, Hydroxy, Carboxy, Trifluormethyl oder Cyan in Frage kommen. Bevorzugt ist der Phenylrest, der durch Halogen (bevorzugt Chlor und Brom), Methoxy, Methyl oder Trifluormethyl substituiert sein kann.

Als Aralkylreste $R_1$ kommen solche in Frage, deren geradkettiger oder verzweigter Alkylenanteil 1-5 Kohlenstoffatome enthält. Bevorzugte Aralkylreste $R_1$ sind der Phenethyl- und der 4-Chlorphenethyl-Rest.

Unter Aralkenylresten $R_1$ sind solche zu verstehen, deren Alkenylenanteil 2-3 Kohlenstoffatome enthält. Hier sind bevorzugt der Styrylrest und der 4-Chlor-styryl-Rest.

Unter den Alkyl-, Alkoxy- und Alkylthiosubstituenten der Aryl-, Aralkyl- und Aralkenylreste sind Reste mit 1-4 C-Atomen bevorzugt, insbesondere die Methyl-, Ethyl-, iso-Butyl- und tert.-Butyl-Gruppe sowie die Methoxy- und die Methylthio-Gruppe.

Unter Halogen ist in allen Fällen Fluor, Chlor und Brom zu verstehen.

n bedeutet bevorzugt die Zahlen 2 bis 4.

$R_3$ bedeutet bevorzugt Wasserstoff oder eine $C_1$-$C_4$-Alkylgruppe, die endständig durch eine Carboxylgruppe oder eine Hydroxygruppe substituiert ist. Die bevorzugte Hydroxyalkylgruppe ist die Hydroxyethylgruppe.

$R_4$ bedeutet bevorzugt ein Wasserstoffatom oder eine $C_{1-4}$ Alkylgruppe, welche ggf. endständig durch Carboxyl oder Hydroxyl substituiert ist. Im letzteren Falle ist auch hier die Hydroxyethylgruppe bevorzugt. Bedeutet $R_4$ einen Phenylrest, so sind besonders bevorzugt der unsubstituierte und der durch ein oder zwei Chloratome substituierte Phenylrest. Bedeutet $R_4$ die Pyridinylgruppe, so ist diese bevorzugt in 2, 3 oder 4-Stellung mit dem Stickstoffatom der $NR_3R_4$-Gruppe verknüpft.

Die Pyridinyl- und die Pyrimidinylgruppe können ggf. mit $C_1$-$C_6$-Alkyl-, $C_1$-$C_6$-Acyl-, Phenyl, Halogen-, Cyano- oder Carboxyl-Substituenten verknüpft sein. Als besonders bevorzugte Cycloalkylreste $R_4$ haben der Cyclopentyl- und der Cylohexylrest zu gelten. Als Acylreste sind Acetyl, Isobutyroyl, Cinnamoyl, Benzoyl, 4-Chlorbenzoyl und 4-Aminobenzoyl, sowie n-Octanoyl und n-Hexadecanoyl bevorzugt.

Bedeutet $R_4$ eine Gruppe

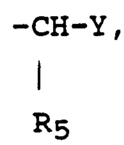

so gilt:

$R_5$ bedeutet bevorzugt ein Wasserstoffatom oder eine unverzweigte oder verzweigte Alkylkette mit 1-4 C-Atomen, welche ggf. endständig substituiert ist.

Als bevorzugte Substituenten sind zu nennen:

Carboxyl, Aminocarbonyl, Mono- bzw. Di-$C_1$-$C_6$-alkylaminocarbonyl, $C_1$-$C_6$-Alkoxycarbonyl, $C_1$-$C_6$-Alkylthio, Phenylthio, Hydroxyl, Phenyl und 4-Imidazolyl.

Y bedeutet bevorzugt eine Carboxylgruppe, kann aber auch $C_1$-$C_6$-Alkoxycarbonyl, Aminocarbonyl, Cyano, Formyl, Hydroxymethyl, Aminomethyl oder eine Orthoestergruppe bedeuten.

Ganz besonders bevorzugt sind solche Verbindungen I, in denen Y = COOH und bei denen $R_3$ und $R_5$ zusammen mit Y = COOH die Struktur einer essentiellen Aminosäuren ergeben. Dazu gehören auch alle möglichen Isomeren und deren Gemische/Razemate.

Zu den Aminosäuren zählen insbesondere Alanin, Arginin, Asparagin, Asparaginsäure, Cystein, Cystin, Glutamin, Glutaminsäure, Glycin, Histidin, Isoleucin, Leucin, Lysin, Methionin, Phenylalanin, Prolin, Serin, Threonin, Tryptophan, Tyrosin, Valin, Homocystein, Homoserin, Hydroxylysin, Hydroxyprolin, Ornithin, Sarkosin, Norvalin oder 2-Aminobuttersäure.

Sind $R_3$ und $R_4$ Bestandteil eines 5- oder 6-gliedrigen gesättigten oder ungesättigten Heterozyclus mit einem oder mehreren Heteroatomen, so kommen für die Gruppe

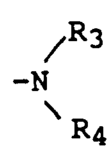

Ringverbindungen in Frage, die über ein Stickstoffatom mit der Alkylkette Q-$(CH_2)_n$- in Formel I verknüpft sind.

Als gesättigte Ringe kommen bevorzugt in Betracht:

Pyrrolidin, Piperidin, Morpholin, Thiomorpholin, 1,1-Dioxothiomorpholin, Piperazin, durch eine Alkyl-, Aralkyl- oder Arylgruppe am Stickstoff substituiertes Piperazin.

Als ungesättigte Ringverbindungen kommen bevorzugt in Frage: Pyrrol, Pyrazol und Imidazol, sowie Benz annellierte Verbindungen, wie Indol, Carbazol oder Verbindungen vom Purin-Typ.

Bevorzugt sind Verbindungen der Formel I, in denen

$R_1$ 4-Chlorphenyl, 4-Methylphenyl, 4-Methoxyphenyl- oder 4-Trifluormethylphenyl,

Q eine Bindung oder Sauerstoff,

n die Zahl, 2, 3 oder 4,

$R_3$ Wasserstoff,

$R_4$ Phenyl, Cyclohexyl, Pyridyl, oder den Rest $-CH_2-COOH$;

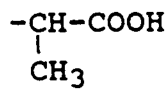

oder

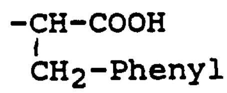

oder $R_3$ und $R_4$ zusammen mit dem Stickstoffatom einen Piperidin-, Piperazin-, Morpholin-, Pyrrol-, Imidazol-, Pyrazol-, Indol- oder Carbazol-Ring bilden.

Für den Fall, daß die Verbindungen der allgemeinen Formel I eine Carboxylfunktion enthalten, kommen als Ester dieser Carbonsäuren solche mit niederen einwertigen Alkoholen (wie z.B. Methanol oder Ethanol) oder mit mehrwertigen Alkoholen (wie z.B. Glycerin) in Frage, es seien aber auch solch Alkohole eingeschlossen, die noch andere funktionelle Gruppen enthalten, wie z.B. Ethanolamin.

Als Amide dieser Carbonsäuren werden solche beansprucht, in denen die Aminkomponente z.B. Ammoniak, ein niederes Dialkylamin wie z.B. Diethylamin oder ein Hydroxyalkylamin wie z.B. Ethanolamin oder Diethanolamin ist. Andere beanspruchte Aminkomponenten sind Alkyl-, Aralkyl- und Arylpiperazine.

Gegenstand der Erfindung sind auch die als Ausgangsmaterial für das Verfahren a) einzusetzenden neuen Carbonamide der allg. Formel II. Verbindungen II, in denen $NR_3R_4$ die Bedeutung einer Aminosäure hat, in denen also $R_3$ = H und $R_4$ = $-CH(R_5)-Y$, sind in der Anmeldung DE-A-3942923.7 beschrieben. Ferner sind nachstehend benannten Verbindungen in EP-A-4011 beschrieben:

1) 4-[2-(Benzoylsulfonamido)ethyl]phenylessigsäure-[4-methylpiperazid]
2) 4-[2-(Benzolsulfonamido)ethyl]phenoxyacetamid
3) 4-[2-(Benzolsulfonamido)ethyl]phenoxyessigsäure-(1-hydroxy-2-propylamid)
4) 4-[2-(Benzolsulfonamido)ethyl]phenoxyessigsäure-(2-carboxyethylamid)
5) 4-[2-(Benzolsulfonamido)ethyl]phenoxyacetanilid und
6) 4-[2-(Benzolsulfonamido)ethyl]phenoxyessigsäure-(4-carboxyanilid)

Die neuen Carbonamide der Formel II werden nach an sich bekannten Verfahren aus der entsprechenden Carbonsäure oder einem reaktiven Derivat derselben durch Umsetzung mit den entsprechenden Aminen hergestellt.

Die Herstellung der neuen Amine der allgemeinen Formel I ist dadurch gekennzeichnet, daß man

a) ein Carbonsäureamid der allgemeinen Formel II

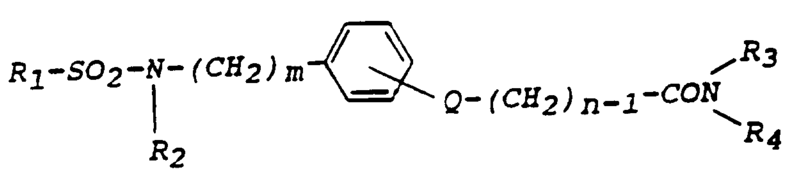 (II),

in welcher $R_1$, $R_2$, $R_3$, $R_4$, Q, m und n die oben angegebene Bedeutung haben, zum Amin reduziert, oder

b) anstelle von Carbonamiden gemäß a) andere, ein Stickstoffatom enthaltende Funktionen, reduziert:

1. Azide III

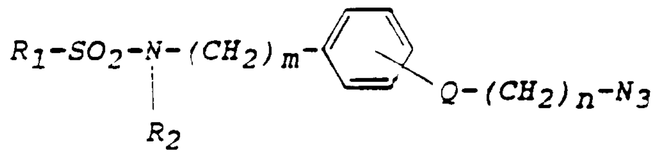

(III),

2. Nitroalkane IV

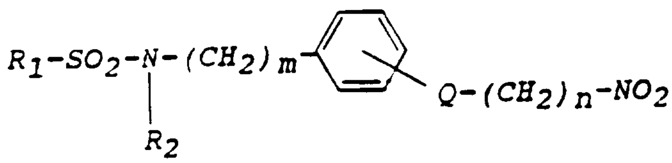

(IV),

3. Nitrile V

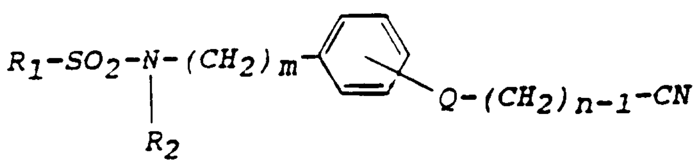

(V),

4. Oxime VI

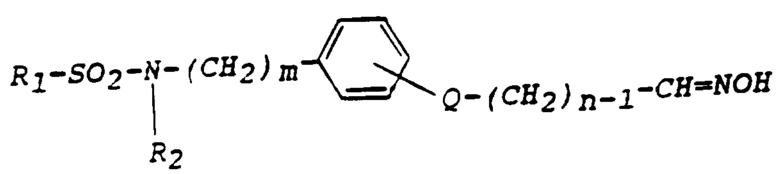

(VI),

wobei jeweils primäre Amine (I, $R_3 = R_4 = H$) entstehen,
oder
5. Schiff-Basen VII

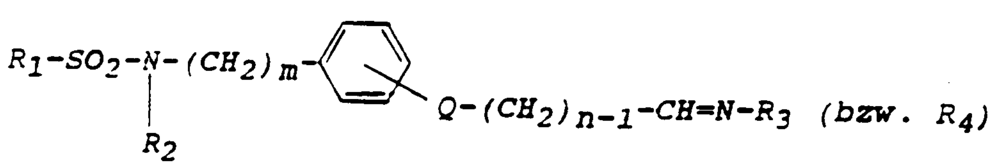

(VII),

wobei sekundäre Amine entstehen
oder

c) eine Verbindung der allgemeinen Formel VIII

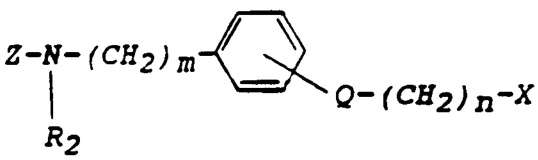

(VIII),

in welcher $R_2$, Q, m und n die oben angebene Bedeutung haben, Z eine Schutzgruppe für die Aminfunktion und X eine reaktive Gruppe darstellen, in an sich bekannter Weise zunächst mit einer gegebenenfalls optisch aktiven Verbindung der allgemeinen Formel IX

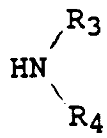

(IX),

($R_3$ und $R_4$ haben auch hier die oben angegebene Bedeutung) und dann, nach Abspaltung der Schutzgruppe Z, mit einer Sulfonsäure der allgemeinen Formel X

$R_1$-$SO_2OH$ (X),

bzw. mit einem Derivat derselben umsetzt,
oder
d) eine Verbindung der allgemeinen Formel XI

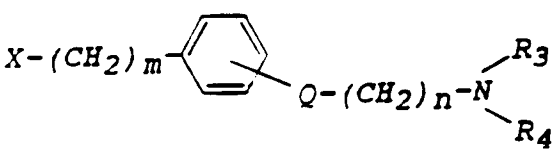

(XI),

mit einem Sulfonamid der allgemeinen Formel XII

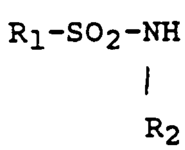

(XII),

zur Umsetzung bringt, wobei $R_1$, $R_2$, $R_3$, $R_4$, X, Q, m und n die oben angegebene Bedeutung haben, oder für den Fall, daß Q = Sauerstoff ist.

e) 1. ein Amin der allgemeinen Formel XIII

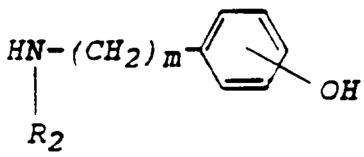

(XIII),

in welcher $R_2$ und m die oben angegebene Bedeutung haben, gegebenenfalls unter intermediärem Schutz der Amino- bzw. Hydroxygruppe, in an sich bekannter Weise in beliebiger Reihenfolge mit einer Sulfonsäure der allg. Formel X bzw. einem Derivat derselben und mit einer gegebenenfalls optisch aktiven Verbindung der allgemeinen Formel XIV

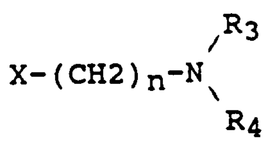

(XIV),

umsetzt, wobei X, n, $R_3$ und $R_4$ haben die oben angegebene Bedeutung haben.

f) Als spezielles Verfahren für die Darstellung von primären Aminen (I, $R_3$ = $R_4$ = H) ist die Delepine-Reaktion zu nennen, welche in der Umsetzung einer Verbindung der Formel XV

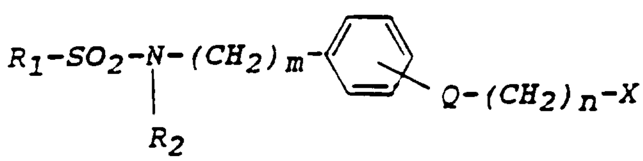

(XV),

in welcher $R_1$, $R_2$, m, n, Q und X die oben angegebenen Bedeutung haben, mit Hexamethylentetramin zu einem quaternären Ammoniumsalz und nachfolgender Hydrolyse besteht.

g) Primäre Amine (I, $R_3$ = $R_4$ = H, Q = Bindung) erhält man auch durch Hofmannschen Abbau von Carbonsäureamiden XVI

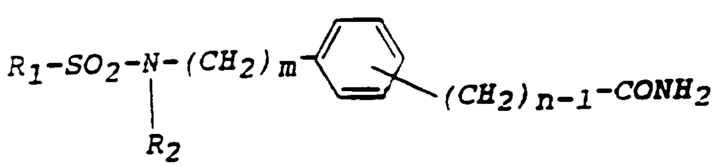

(XVI)

mittels Alkalihypobromit.

h) Als spezielles Verfahren für die Herstellung von primären Phenethylaminen (I, Q = Bindung, n = 2, $R_3$ = $R_4$ =H) ist die Reduktion von Nitrostyrolen XVII

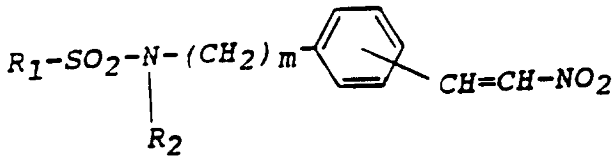

(XVII)

zu nennen.

i) Primäre Amine erhält man, indem man in Verfahren a) an Stelle von II ein geschütztes Carbonamid der allgemeinen Formel IIa

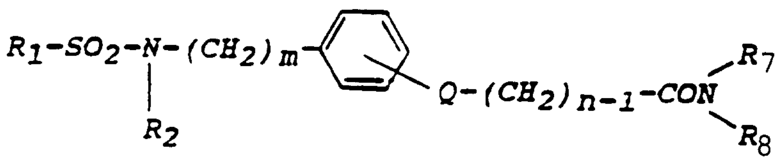

(IIa),

in welcher $R^1$, $R^2$, m, Q, n die oben angegebenen Bedeutungen haben und $R^7$ = H, $R^8$ = eine Schutzgruppe oder $R^7$ und $R^8$ gemeinsam eine Schutzgruppe bedeuten, einsetzt und nach erfolgter Reduktion die Schutzgruppe entfernt.

j) Primäre Amine erhält man auch, indem man in Verfahren e) anstelle von XIV ein geschütztes Amin der allgemeinen Formel XIVa

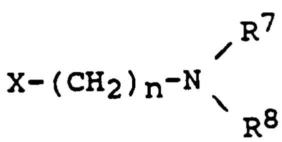

(XIVa),

in welcher X, n und $R^7$, $R^8$ die oben angegebene Bedeutung haben, einsetzt und anschließend die Schutzgruppe entfernt.

Für die Reduktion der Carbonamide gemäß Verfahren a) bzw. der Azide, Nitro- und Cyanoverbindungen, Oxime bzw. Schiff-Basen gemäß Verfahren b) können im Prinzip alle für die jeweiligen Gruppen literaturbekannten Verfahren angewandt werden. Als Reduktionsmittel bieten sich an: Komplexe Bor- und Aluminiumhydride, Borwasserstoff-Komplexe, Aluminiumhydrid ($LiAlH_4$ + $AlCl_3$), ein Gemisch aus $AlCl_3$ und $NaBH_4$, Wasserstoff in statu nascendi (Alkohol + Natrium) sowie katalytisch angeregter Wasserstoff, ggf. unter Druck. Enthalten die zu reduzierenden Carbonamide II solche Gruppen $R^3$ bzw. $R^4$, welche mit starken Reduktionsmitteln ebenfalls verändert wurden (z. B. Alkoxycarbonyl), so wandelt man das Carbonamid zunächst mit $PCl_5$ in einen "Vilsmeier-Komplex" um und reduziert diesen dann mit einem milde wirkenden Reduktionsmittel wie Natriumborhydrid oder Zink zu Ethanol.

Mit $NaBH_4$ tritt allerdings häufiger eine partielle Reduktion der Alkoxycarbonylgruppe zur Alkoholfunktion auf.

Die genannten Reduktionsverfahren gelten auch für den Fall, daß man gemäß i) geschützte Carbonamide reduzieren will.

Die bei Verfahren b) eingesetzten Ausgangsverbindungen sind zum Teil neu und können nach an sich bekannten Verfahren aus entsprechenden Alkoholen oder Aldehyden erhalten werden. Die Nitrile der Formel II sind z. B. teilweise in der EP-A-356 989 beschrieben und können nach den angeführten Verfahren hergestellt werden.

Die Umsetzungen einer Verbindung der allgemeinen Formel VIII mit einem Amin der allg. Formel IX (d.h. Verfahren c) erfolgt zweckmäßig so, daß man zunächst die Aminogruppe einer Verbindung der allg. Formel XVIII

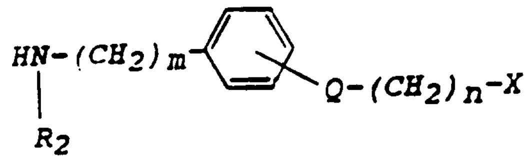

(XVIII),

in der $R_2$, m, Q, n und X die oben angegebene Bedeutung haben mit einer leicht wieder abspaltbaren Schutzgruppe blockiert, so daß eine Verbindung VIII entsteht. Besonders geeignet sind hier die aus der Peptidchemie bekannten, durch Hydrierung oder durch saure Hydrolyse leicht zu entfernenden Schutzgruppen wie z.B. die Benzyloxycarbonylgruppe. Geeignet sind auch Schutzgruppen wie die Phthalimidogruppe, die nach erfolgter Kondensation zwischen VIII und IX z.B. mittels Hydroxylamin leicht wieder abgespalten werden kann. Gelegentlich kann man auf die Wiederabspaltung ganz verzichten, indem man von vornherein die Gruppe $R_1$-$SO_2$- einführt. Als reaktive Gruppen X der Verbindungen VIII (wie auch aller folgenden, X enthaltenden Verbindungen) kommen insbesondere solche infrage, bei denen X ein Halogenatom oder eine Alkylsulfonyloxy- bzw. Arylsulfonyloxygruppe darstellt. Die Umsetzung einer Verbindung VIII mit einer Verbindung IX erfolgt zweckmäßig in inerten Lösungsmitteln wie z.B. Toluol oder Methylenchlorid, wobei man als Säureakzeptor zweckmäßig einen Überschuß eines tert. Amins wie z.B. Pyridin oder Triethylamin zusetzt.

Die Abspaltung der Schutzgruppe erfolgt nach Verfahren, wie sie aus der Peptidchemie literaturbekannt sind. So läßt sich die Benzyloxycarbonylgruppe z.B. durch katalytische Hydrierung, aber auch durch saure Hydrolyse z.B. mit wäßriger HBr-Lösung, abspalten. Die daraus resultierenden Verbindungen der allgemeinen Formel XIX

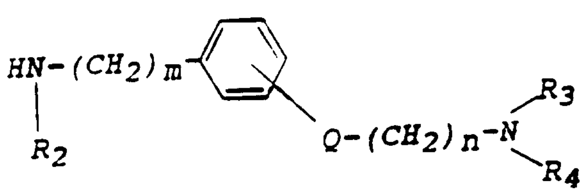

(XIX),

in der $R_2$, $R_3$, $R_4$, m, n und Q die oben angegebene Bedeutung haben, werden nun in Sulfonamide überführt. Als reaktive Derivate der Sulfonsäuren X kommen insbesondere deren Halogenide sowie deren Ester in Betracht. Die Umsetzungen der bevorzugt angewandten Sulfonsäurechloride mit den Verbindungen XIX erfolgen zweckmäßig unter Zusatz eines säurebindenden Mittels, wie z.B. Alkaliacetat, Alkalicarbonat, Alkalihydrogencarbonat, Natriumphosphat, Alkalihydroxid, Calciumoxid, Calciumcarbonat oder Magnesiumcarbonat. Diese Funktion können aber auch organische Basen wie z.B. Pyridin oder Triethylamin übernehmen, wobei als inertes Lösungsmittel z.B. Ether, Methylenchlorid, Dioxan, Toluol oder ein Überschuß des tertiären Amins dient. Beim Einsatz anorganischer Säurebinder verwendet man als Reaktionsmedium z.B. Wasser, wäßriges Ethanol oder wäßriges Dioxan.

Für die unter d) beschriebene Umsetzung zwischen einer Verbindung XI mit einem Sulfonamid XII hat es sich als besonders vorteilhaft erwiesen, ein primäres Sulfonamid XII, $R_2$ = H zunächst durch Umsetzung z.B. mit Hexamethyldisilazan in das Trimethylsilylsulfonamid XII, hier $R_2$ = $-SiMe_3$, umzusetzen.

Dessen Umsetzung mit XI liefert ein Produkt, welches frei von disubstituiertem Sulfonamid ist. Ein anderer Weg bedient sich der Alkalisalze der Sulfonamide XII: Zwei Mol Sulfonamid XII werden mit einem Mol alkoholischer Natriumalkoholat-Lösung zur Trockne eingedampft. Das erhaltene Gemisch wird nun mit einem Mol einer Verbindung XI umgesetzt. Auch so läßt sich die Kondensation des Sulfonamids mit zwei Mol XI verhindern.

Das unter e) skizzierte Verfahren führt man zweckmäßig in zwei Stufen durch. Die Kondensation der Verbindungen der allgemeinen Formel XIII mit Sulfonsäuren (X) oder deren Derivaten einerseits und Verbindungen der allgemeinen Formel XIV andererseits wird vorzugsweise so durchgeführt, daß man zunächst eine der beiden reaktiven Gruppen der Verbindung XIII mit einer leicht abspaltbaren Schutzgruppe blockiert, die erhaltene Verbindung mit einer Sulfonsäure (X) oder einem Derivat davon bzw. mit einer Verbindung der allgemeinen Formel XIV umsetzt, die Schutzgruppe wieder abspaltet und anschließend dieses reaktive Zwischenprodukt mit der noch nicht eingesetzten Verbindung der allgemeinen Formel XIV

bzw. (X) umsetzt. Bevorzugt wird ein Verfahren, bei dem die an der Aminogruppe geschützte Verbindung XIII (d.h. die Verbindung XX) zunächst mit einer Verbindung XIV zur Umsetzung gebracht wird. Nach Abspaltung der Schutzgruppe erfolgt dann die Reaktion mit einer Sulfonsäure (X) bzw. mit einem ihrer Derivate:

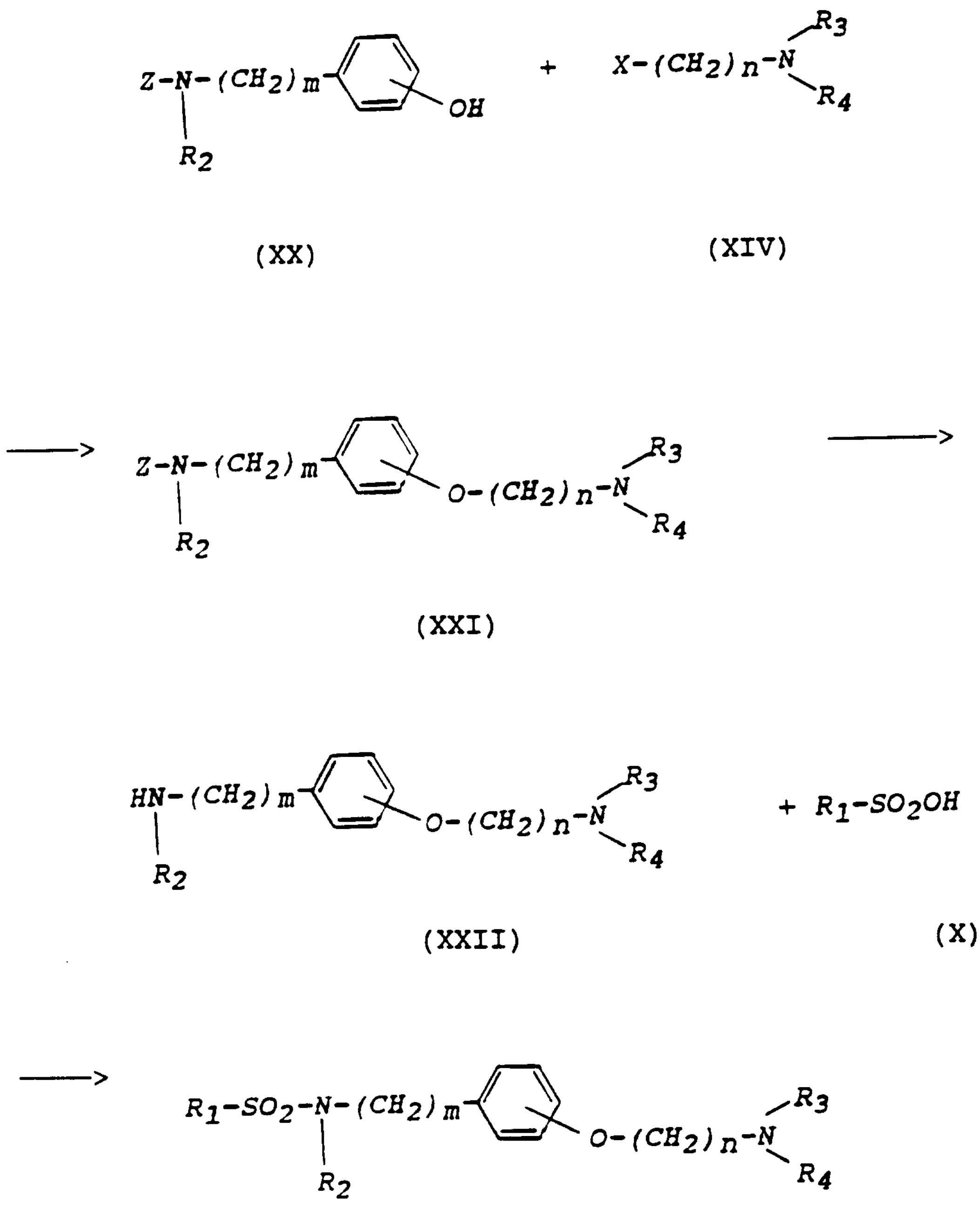

(XX) (XIV)

(XXI)

(XXII) (X)

I, Q = O

Die in den oben stehenden Formeln verwendeten Symbole haben die bereits angegebene Bedeutung.

Eine analoge Verfahrensweise wird angewendet, wenn man zu solchen Verbindungen I, Q = 0 kommen will, die an der Aminogruppe nachträglich modifiziert werden sollen:

Man setzt anstelle von XIV eine Verbindung der allgemeinen Formel XIVa

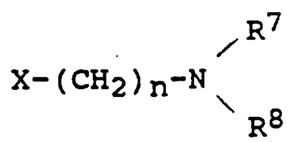

(XIVa),

in welcher X, n und $R^7$, $R^8$ die unter j) angegebene Bedeutung haben, mit einer Verbindung XX um, gelangt zu einer Verbindung XXIa (in der die Substituenten $R_3$ und $R_4$ der Verbindung XXI durch $R_7$, $R_8$ ersetzt sind), entfernt die Schutzgruppe Z (wobei XXIIa entsteht) und erhält nach Umsetzen mit einer Sulfonsäure (X) eine Verbindung Ia, Q = 0:

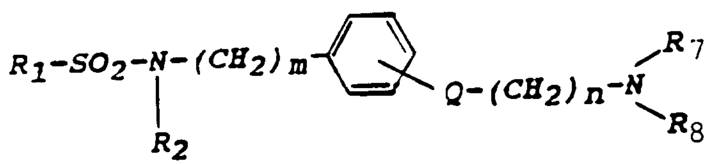

(Ia).

Diese Verbindung kann nach Entfernen der Schutzgruppe $R^7$ bzw. $R^7$, $R^8$ für zahlreiche Umwandlungen an der Aminogruppe ($R^7$, $R^8$ = H) benutzt werden.

Die Reaktion eines Phenols XX mit einer aktivierten Verbindung XIV bzw. XIVa wird zweckmäßig so ausgeführt, daß man das Phenol in Form seines Natrium- oder Kaliumsalzes einsetzt. Als Reaktionsmedium dienen Lösungsmittel wie z.B. Toluol, Methylethylketon, Dimethylformamid oder Dimethylsulfoxid.

Die gegebenenfalls nachträgliche N-Alkylierung einer Verbindung der allgemeinen Formel (I), kann nach bekannten Methoden durchgeführt werden, vorzugsweise, indem man sie mit einem Alkylhalogenid oder einem Dialkylsulfat in Gegenwart eines säurebindenden Mittels wie z.B. Kaliumcarbonat umsetzt. Für den Fall, daß $R_3$ oder/und $R_4$ ein Wasserstoffatom bedeuten, müssen diese Wasserstoffatome intermediär durch eine Schutzgruppe ersetzt werden. Als Schutzgruppen dienen auch hier die aus der Peptidchemie bekannten, z.B. die Benzyloxycarbonylgruppe oder $NR^3R^4$ bedeutet z. B. eine Phthalimidogruppe.

Für den Fall, daß $R_4$ eine Gruppe $-CH(R_5)COOH$ bedeutet, lassen sich Verbindungen der allgemeinen Formel I besonders gut darstellen, indem an Stelle von Reaktionskomponenten mit freier Carboxylgruppe solche mit "maskierter Carboxylfunktion" einsetzt. Man erhält dann zunächst Verbindungen der allgemeinen Formel I, in welcher $R_4$ eine Gruppe $-CH(R_5)-Y$ darstellt, wobei Y die Bedeutung Alkoxycarbonyl, Aminocarbonyl, Cyano, Formyl, Hydroxymethyl, Aminomethyl oder einer Orthoestergruppe haben kann. Die Umwandlung dieser Gruppen in die Carboxylfunktion erfolgt dann nach literaturbekannten Verfahren durch Hydrolyse (Alkoxycarbonyl, Cyano, Orthoester) oder Oxidation (Formyl, Hydroxymethyl, Aminomethyl).

Umgekehrt kann man aber auch die Carbonsäuren I, $R_4$ = $-CH(R_5)-COOH$ in üblicher Weise verestern, wobei Verbindungen I, $R_4$ = $-CH(R_5)-COOR_6$ entstehen (zum Umfang der Bedeutung von $R_6$ siehe weiter unten) oder deren Ester mit einem bestimmten Rest R durch Umestern in einen Ester mit einem anderen Rest R umwandeln. Die Veresterung der Carbonsäuren wird zweckmäßig in Gegenwart eines sauren Katalysators wie z.B. Chlorwasserstoff, Schwefelsäure, p-Toluolsulfonsäure, oder eines stark sauren Ionenaustauschharzes, vorgenommen. Umesterungen hingegen erfordern den Zusatz einer geringen Menge einer basischen Substanz, z.B. eines Alkali- oder Erdalkalihydroxids oder eines Alkalialkoholats. Für die Veresterung der Carboxygruppe bzw. für eine Umesterung eignen sich prinzipiell alle Alkohole. Bevorzugt sind die niederen einwertigen Alkohole wie Methanol, Ethanol oder Propanol, sowie mehrwertige Alkohole, z.B. Glycerin oder Alkohole mit anderen funktionellen Gruppen, wie Ethanolamin oder Glycerinether.

Die erfindungsgemäßen, von den Carbonsäuren der allgemeinen Formel I, $R_4$ = $CH(R_5)COOH$ abgeleiteten Amide werden bevorzugt nach an sich bekannten Methoden aus den Carbonsäuren oder ihren reaktiven Derivaten (wie z.B. Carbonsäurehalogeniden, -estern, -aziden, -anhydriden oder gemischten Anhydriden) durch Umsetzung mit Aminen hergestellt. Als Aminokomponenten kommen z.B. Ammoniak, Alkylamine, Dialkylamine, aber auch Aminoalkohole wie z.B. Ethanolamin und 2-Aminopropanol infrage. Andere wertvolle Aminkomponenten sind Alkyl-, Aralkyl- und Arylpiperazine.

Zur Herstellung von Salzen mit pharmakologisch verträglichen organischen oder anorganischen Basen, wie z.B. Natriumhydroxid Kaliumhydroxid, Calciumhydroxid, Ammoniumhydroxid, Methylglukamin, Morpholin oder Ethanolamin können die Carbonsäuren mit den entsprechenden Basen umgesetzt werden. Auch Mischungen der Carbonsäuren mit einem geeigneten Alaklicarbonat bzw. -hydrogencarbonat kommen in Betracht.

Zu reinen Enantiomeren der Verbindungen der Formel I kommt man entweder durch Razematspaltung (über Salzbildung mit optisch aktiven Basen), oder, indem man in die Synthesen gemäß Verfahren c) und d-f) jeweils optisch reine Amine/Aminosäuren einsetzt.

Zur Herstellung von Arzneimitteln werden die Verbindungen der allgemeinen Formel I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, wie z.B. Olivenöl, suspendiert oder gelöst.

Die Substanzen der allgemeinen Formel I können in flüssiger oder fester Form oral und parenteral appliziert werden. Als Injektionsmedium kommt vorzugsweise Wasser zur Anwendung, welches die bei Injektionslösungen üblichen Stabilisierungsmittel, Lösungsvermittler und/oder Puffer enthält. Derartige Zusätze sind z.B. Tartrat- oder Borat-Puffer, Ethanol, Dimethylsulfoxid, Komplexbildner (wie Ethylendiamintetraessigsäure), hochmolekulare Polymere (wie flüssiges Polyethylenoxid) zur Viskositätsregulierung, oder Polyethylen-Derivate von Sorbitanhydriden.

Feste Trägerstoffe sind z.B. Stärke, Lactose, Mannit, Methylcellulose, Talkum, hochdisperse Kieselsäure, höhermolekulare Fettsäuren (wie Stearinsäure), Gelatine, Agar-Agar, Calciumphosphat, Magnesiumstearat, tierische und pflanzliche Fette, oder feste hochmolekulare Polymere (wie Polyethylenglykole). Für die orale Applikation geeignete Zubereitungen können gewünschtenfalls Geschmacks- und Süßstoffe enthalten.

Bevorzugt im Sinne der Erfindung sind außer den in den Beispielen genannten Verbindungen der Formel I die folgenden:

1) 1-[2-[2-(4-Chlor-phenylsulfonylamino)ethyl]phenoxy]-2-aminoethan
2) 1-[3-[2-(4-Chlor-phenylsulfonylamino)ethyl]phenoxy]-3-aminopropan
3) 1-[3-[2-(4-Chlor-phenylsulfonylamino)ethyl]phenoxy]-4-aminobutan; Hydrochlorid, Schmp. 153 ° C
4) 1-[3-[2-[4-Methyl-phenylsulfonylamino)ethyl]phenoxy]-2-aminoethan, Hydrochlorid, Schmp. 128-130 ° C
5) 1-[3-[2-(Trifluormethyl-phenylsulfonylamino)ethyl]phenoxy]-2-aminoethan
6) 1-[4-[2-(4-Chlor-phenylsulfonylamino)ethyl]phenoxy]-4-aminobutan
7) 1-[4-[2-(4-Methyl-phenylsulfonylamino)ethyl]phenoxy]-2-piperidinoethan
8) 1-[3-[2-(4-Methyl-phenylsulfonylamino)ethyl]phenoxy]-2-piperidinoethan
9) 1-[3-[2-(4-Chlor-phenylsulfonylamino)ethyl]phenoxy]-2-anilinoethan; Hydrochlorid, Schmp. 124-126 ° C
10) 1-[3-[2-(4-Chlor-phenylsulfonylamino)ethyl]phenoxy]-2-(cyclohexylamino)ethan; Hydrochlorid, Schmp. 150-151 ° C
11) N-[2-[3-[2-(4-Chlor-phenylsulfonyl-amino)ethyl]phenoxy]ethyl]glycin
12) N-[2-[3-[2-(4-Chlor-phenylsulfonyl-amino)ethyl]phenoxy]ethyl]-L-alanin
13) N-[2-[4-[2-(4-Chlor-phenylsulfonyl-amino)ethyl]phenoxy]ethyl]-L-alanin
14) N-[2-[3-[2-(4-Methyl-phenylsulfonyl-amino)ethyl]phenoxy]ethyl]-L-alanin
15) N-[2-[3-[2-(4-Chlor-phenylsulfonyl-amino)ethyl]phenoxy]ethyl]phenylalanin
16) N-[4-[2-(4-Chlor-phenylsulfonylamino)ethyl]benzyl]glycin
17) N-[4-[2-(4-Chlor-phenylsulfonylamino)ethyl]benzyl]-L-alanin
18) N-[4-[2-(4-Chlor-phenylsulfonylamino)ethyl]phenethyl]glycin
19) N-[4-[2-(4-Chlor-phenylsulfonylamino)ethyl]phenethyl]-L-alanin
20) 1-[4-[2-(4-Chlor-phenylsulfonylamino)ethyl]phenoxy]-2-(3-pyridylamino)ethan
21) 1-[4-[2-(4-Chlor-phenylsulfonylamino)ethyl]phenyl]-2-(4-pyridylamino)ethan
22) 1-[4-[2-(4-Chlor-phenylsulfonylamino)ethyl]phenyl]-2-(2-pyridylamino)ethan
24) N-[2-[4-[2-(4-Chlor-phenylsulfonylamino)ethyl]phenyl]ethyl]pyrrol
25) N-[2-[4-[2-(4-Chlor-phenyl-sulfonyl)ethyl]phenoxy]ethyl]pyrrol
26) N-[2-[4-[2-(4-Chlor-phenylsulfonyl-amino)ethyl]phenyl]ethyl]imidazol
27) N-[2-[4-[2-(4-Chlor-phenylsulfonyl-amino)ethyl]phenoxy]ethyl]imidazol
28) N-[2-[4-[2-(4-Chlor-phenylsulfonyl-amino)ethyl]phenyl]ethyl]pyrazol
29) N-[2-[4-[2-(4-Chlor-phenylsulfonyl-amino)ethyl]phenoxy]ethyl]pyrazol
30) N-[2-[4-[2-(4-Chlor-phenylsulfonyl-amino)ethyl]phenyl]ethyl]indol
31) N-[2-[4-[2-(4-Chlor-phenylsulfonyl-amino)ethyl]phenoxyindol
32) N-[2-[4-[2-(4-Chlor-phenylsulfonyl-amino)ethyl]phenyl]ethyl]carbazol
33) 1-[4-[2-(4-Chlor-phenylsulfonylamino)ethyl]phenoxy]-2-(2-pyridinylamino)ethan
34) 1-[4-[2-(4-Chlor-phenylsulfonylamino)ethyl]phenoxy]-2-(3-pyridinylamino)ethan
35) 1-[4-[2-(4-Chlor-phenylsulfonylamino)ethyl]phenoxy]-2-(4-pyridinylamino)ethan

36) 1-[3-[2-(4-Chlor-phenylsulfonylamino)ethyl]phenoxy]-2-(2-pyridinylamino)ethan
37) 1-[3-[2-(4-Chlor-phenylsulfonylamino)ethyl]phenoxy]-2-(3-pyridinylamino)ethan
38) 1-[3-[2-(4-Chlor-phenylsulfonylamino)ethyl]phenoxy]-2-(4-pyridinylamino)ethan
39) 1-[4-[2-(4-Chlor-phenylsulfonylamino)ethyl]phenyl]-2-(2-pyridinylamino)ethan
40) 1-[4-[2-(4-Chlor-phenylsulfonylamino)ethyl]phenyl]-2-(3-pyridinylamino)ethan
41) 1-[4-[2-(4-Chlor-phenylsulfonylamino)ethyl]phenyl]-2-(4-pyridinylamino)ethan

Ebenfalls bevorzugt sind neue Carbonamide der Formel II als Zwischenprodukte, die zu den bevorzugten Verbindungen No. 1-10 und 20-41 führen, sowie die in den Beispielen 1-3 beschriebenen Verbindungen.

Die nachfolgenden Beispiele zeigen einige der zahlreichen Verfahrensvarianten, die zur Synthese der erfindungsgemäßen Verbindungen verwendet werden können. Sie sollen jedoch keine Einschränkung darstellen.

Vorstufen: Carbonsäureamide der allg. Formel II

Beispiel 1

2-[4-[2-(4-Chlor-phenylsulfonylamino)ethyl]phenyl]essigsäurepiperidid

Ein Gemisch aus 10.0 g (28 mmol) 2-[4-[2-(4-Chlor-phenylsulfonylamino)ethyl]phenyl]essigsäure, 10 ml Thionylchlorid und 3 Tropfen DMF wird zwei Stdn. bei 60 °C gerührt. Dann destilliert man überschüssiges Thionylchlorid i. Vak. ab und löst den Rückstand in 50 ml Methylenchlorid. Diese Lösung tropft man unter Eiskühlung zu einer Lösung aus 7.22 g (85 mmol) Piperidin und 80 ml Methylenchlorid. Anschließend wird eine Std. lang unter Eiskühlung, dann eine Std. bei Raumtemperatur gerührt. Dann schüttelt man nacheinander zweimal mit 2 N HCl, zweimal mit Wasser und zweimal mit $NaHCO_3$-Lösung aus. Dann trocknet man über Magnesiumsulfat, dampft ein und kristallisiert aus Essigester um. Ausb.: 7.2 g (61 %); Schmp. 110 °C.

In Analogie dazu wurde hergestellt:

a) 3-[4-[2-(4-Chlor-phenylsulfonylamino)ethyl]phenyl]propionamid
aus 3-[4-[2-(4-Chlor-phenylsulfonylamino)ethyl]phenyl] propionsäure, $SOCl_2$ und Ammoniak. Das Säurechlorid wurde in Methylenchlorid gelöst, dann mit Ammoniak begast.
Ausb. 77 % d. Th.; Schmp. 169-170 °C (Methanol).

b) 4-[2-(4-Chlor-phenylsulfonylamino)ethyl]phenylessigsäureamid
aus 4-[2-(4-Chlor-phenylsulfonylamino)ethyl]phenylessigsäure, $SOCl_2$ und Ammoniak.
Ausb. 76 % d. Th.; Schmp. 192-193 ° C.

c) 4-[2-(4-Chlor-phenylsulfonylamino)ethyl-phenylessigsäure-ethylamid
aus 4-[2-(4-Chlor-phenylsulfonylamino)ethyl]phenylessigsäure, $SOCl_2$ und Ethylamin.
Ausb. 62 % d. Th.; Schmp. 113 ° C (wäßr. Ethanol).

Aus 3-[2-(4-Chlor-phenylsulfonylamino)ethyl]phenoxyessigsäure, $SOCl_2$ und dem entsprechenden Amin wurden außerdem dargestellt:

d) 3-[2-(4-Chlor-phenylsulfonylamino)ethyl]phenoxyacetylpiperidid
Ausb. 77 % d.Th.; Schmp. 124-126 °C (Ethanol)

e) 3-[2-(4-Chlor-phenylsulfonylamino)ethyl]phenoxyacetyldiethylamid
Ausb. 87 % d.Th.; Schmp. 78-80 °C (Essigester + Isohexan)

f) 3-[2-(4-Chlor-phenylsulfonylamino)ethyl]phenoxyessigsäure-(2-hydroxyethylamid)
Ausb. 65 % d.Th.; farbl. Öl.

g) 3-[2-(4-Chlor-phenylsulfonylamino)-ethyl]phenoxyessigsäure]-2-[bis(2-hydroxyethyl)amid]
Ausb. 66 % d.Th.; farbl. Öl.

h) 3-[2-(4-Chlor-phenylsulfonylamino)ethyl]phenoxyessigsäure-morpholid
Ausb. 86 % d.Th.; Schmp. 110-111 °C (Ethanol).

i) 3-[2-(4-Chlor-phenylsulfonylamino)ethyl]phenoxyessigsäure-(4-methyl-piperazid)
Ausb. 98 % d. Th.; Schmp. 139-141 °C (Ethanol).

j) 3-[2-(4-Chlor-phenylsulfonylamino)ethyl]phenoxyessigsäure-anilid
Ausb. 89 % d. Th.; Schmp. 143-144 °C.

k) 3-[2-(4-Chlor-phenylsulfonylamino)ethyl]phenoxyessigsäure-cyclohexylamid
Ausb. 82 % d. Th.; Schmp. 100-101 °C.

Beim Einsatz von 3-[2-(3-Trifluormethyl-phenylsulfonylamino)ethyl]phenoxyessigsäure bzw. von 3-[2-(4-Brom-phenylsulfonylamino)ethyl]phenoxyessigsäure erhält man mit $SOCl_2$ und Ammoniak die Verbindungen:

l) 3-[2-(3-Trifluormethyl-phenylsulfonylamino)ethyl]phenoxyacetamid
Ausb. 71 % d. Th.; Schmp. 118 °C (Ethanol).
m) 3-[2-(4-Brom-phenylsulfonylamino)ethyl]phenoxyacetamid
Ausb. 81 % d. Th.; Schmp. 115-116 °C (Ethanol).

Aus 4-[2-(4-Chlor-phenylsulfonylamino)ethyl]phenoxyessigsäure, $SOCl_2$ und em entsprechenden Amin erhält man:

n) 4-[2-(4-Chlor-phenylsulfonylamino)ethyl]phenoxyessigsäuredietyhlamid
Ausb. 80 % d. Th.; farbloses Öl.
o) 4-[2-(4-Chlor-phenylsulfonylamino)ethyl]phenoxyessigsäure-ethylamid
Ausb. 81 % d. Th.; Schmp. 133-134 °C.
p) 4-[2-(4-Chlor-phenylsulfonylamino)ethyl]phenoxyessigsäure-(4-methyl-piperazid)
Ausb. 78 % d. Th.; Schmp. 110-112 °C.
q) 4-[2-(4-Chlor-phenylsulfonylamino)ethyl]phenoxyessigsäure-(2-pyrimidinyl)amid
Ausb. 60 % d. Th.; Hydrochlorid Schmp. 150-157 °C.
r) 4-[2-[4-Chlor-phenylsulfonylamino)ethyl]phenoxyessigsäure-(3-pyridyl)amid
Ausb. 50 % d. Th.; Hydrochlorid Schmp. 152-155 °C.

## Beispiel 2

4-[3-[2-(4-Chlor-phenylsulfonylamino)ethyl]phenoxy]butyramid

Zu einer auf -10 °C abgekühlten Lösung aus 100 ml abs. THF, 4.32 g (43 mmol) Triethylamin und 14.4 g (36 mmol) 4-[3-[2-(4-Chlor-phenylsulfonylamino)ethyl]phenoxy]buttersäure tropft man langsam ein Gemisch aus 4.02 g (37 mmol) Chlorameisensäureethylester und 30 ml abs. THF. Man läßt auf Raumtemperatur kommen, rührt weitere 30 min und saugt ausgefallenes Triethylamin-Hydrochlorid ab. Das Filtrat wird nun unter Eiskühlung mit Ammoniak begast. Dann wird 6 Stdn. bei Raumtemperatur gerührt, eingedampft und mit 2 N HCl versetzt. Man gibt Essigester zu, durchmischt sehr heftig und trennt die Phasen. Die Essigesterphase wird zweimal mit Bicarbonatlösung und zweimal mit Wasser ausgeschüttelt, dann mit $Na_2SO_4$ getrocknet und eindampft. Nach Umkristallisieren aus Essigester erhält man farblose Kristalle, Ausb.: 12.8 g (89 % d.Th.); Schmp. 110-112 °C.

In analoger Weise lassen sich darstellen:

a) 3-[2-(4-Chlor-phenylsulfonylamino)ethyl]phenoxyessigsäure-ethylamid
Ausb. 83 % d. Th.; farbloses Öl.
b) 4-[2-(4-Chlor-phenylsulfonylamino)ethyl]phenylessigsäure-(4-pyridyl)amid
Ausb. 21 % d. Th.; Hydrochlorid Schmp. 181 °C

Eine Variante des Verfahrens besteht in der Verwendung eines aktiven Nitrophenylesters zur Amidbildung:

c) 3-[2-(4-Methyl-phenylsulfonylamino)ethyl]phenoxyessigsäure-piperidid
Zu einer 40 °C warmen Lösung aus 12,0 g (34 mmol) 3-[2-(4-Methyl-phenylsulfonylamino)ethyl]-phenoxyessigsäure und 120 ml abs. THF gibt man 5,6 g (34 mmol) Carbonylbisimidazol, läßt 15 min. reagieren, setzt 0,5 g (3,4 mmol) 4-Nitrophenol zu, läßt wiederum 15 min. reagieren und gibt nun 2,92 g (34 mmol) Piperidin zu. Nun wird zwei Stunden bei 60 °C gehalten. Dann dampft man ein, versetzt den Rückstand mit einem Eiswasser-Salzsäuregemisch bis zur deutlich sauren Reaktion und extrahiert dreimal mit Essigester. Der Extrakt wird dreimal mit Wasser, zweimal mit 2N-$Na_2CO_3$-Lösung und einmal mit Wasser gewaschen, dann trocknet man ($Na_2SO_4$) und dampft ein.
Ausb. 10,4 g (73 % d. Th.), Schmp. 119-120 °C (Ethanol).

In zu c) analoger Weise wurde aus 4-[2-(4-Chlor-phenylsulfonylamino)ethylbenzoesäure und Piperidin dargestellt:

d) 4-[2-(4-Chlor-phenylsulfonylamino)ethyl]benzoesäurepiperidid
Ausb. 62 % d. Th.; Schmp. 190 °C

Und aus 3-[4-[2-(4-Chlor-phenylsulfonylamino)ethyl]phenyl]propionsäure und Piperidin wird dargestellt:

e) 3-[4-[2-(4-Chlor-phenylsulfonylamino)ethyl]phenyl]propionsäure-piperidid
Ausb. 71 % d. Th.; Schmp. 89-91 °C.

Beispiel 3

4-[2-[4-Chlor-phenylsulfonylamino)ethyl]phenoxyessigsäure-[bis(2-hydroxyethyl)amid]

Man hält ein Gemisch aus 9.44 g (25 mmol) 4-[2-(4-Chlor-phenylsulfonylamino)ethyl]-phenoxyessigsäure-ethylester, 30 ml Methanol und 30 ml Diethanolamin fünf Stdn. auf Rückflußtemperatur und dampft anschließend ein. Der Rückstand wird mit 300 ml Essigsäureethylester versetzt. Man rührt diese Lösung mit so viel 2 N HCl, daß die wäßrige Phase sauer reagiert. Sie wird abgetrennt, die Essigesterphase wird mit Wasser neutral gewaschen, getrocknet ($Na_2SO_4$) und schließlich eingedampft.
Ausb. 9.1 g (80 % d.Th.); farbl. Öl.
In analoger Weise wurden aus

4-[2-(4-Chlor-phenylsulfonylamino)ethyl]phenoxyessigsäureethylester und dem entsprechenden Amin dargestellt:

a) 4-[2-(4-Chlor-phenylsulfonylamino)ethyl]phenoxyessigsäure-(2-hydroxyethylamid)
Ausb. 91 %; Schmp. 112-114 °C
b) 4-[2-(4-Chlor-phenylsulfonylamino)ethyl]phenoxyessigsäure-piperidid
Ausb. 93 % d.Th.; Schmp. 124-126 °C
c) 4-[2-(4-Chlor-phenylsulfonylamino)ethyl]phenoxyessigsäuremorpholid
Ausb. 91 % d.Th.; farbl. Öl.
d) 4-[2-(4-Chlor-phenylsulfonylamino)ethyl]phenoxyessigsäureamid
Der Ester wird in Methanol gelöst. Man gibt in der Kälte flüssiges $NH_3$ zu, läßt bei Raumtemperatur überschüssiges $NH_3$ abdampfen und saugt ausgefallenes Produkt ab.
Ausb. 88 % d.Th.; Schmp. 158-159 °C.

Amine der allg. Formel I

Beispiel 4

1-[3-[2-(4-Chlor-phenylsulfonylamino)ethyl]phenoxy]-2-aminoethan

Zu einer Suspension aus 1.55 g (41 mmol) $LiAlH_4$ und 200 ml abs. THF tropft man bei Raumtemperatur unter Rühren eine Lösung aus 10.0 g (27 mmol) 3-[2-(4-Chlor-phenylsulfonylamino)ethyl]phenoxyacetamid (Darst. in EP 89115 911.3 Bsp. 14 beschrieben) und 150 ml abs. THF. Das Gemisch wird zwei Stdn. auf Rückflußtemperatur gehalten, dann abgekühlt und mit Eiswasser zersetzt. Man saugt ab und dampft das Filtrat ein. Das zurückbleibende Öl wird in Methylenchlorid aufgenommen. Man trocknet ($MgSO_4$), löst in Ether und fällt durch Zugabe von Chlorwasserstoff enthaltendem Ether das Hydrochlorid. Nach Umkristallisieren aus Ethanol erhält man farblose Kristalle,
Ausbeute: 9.3 g (88 % d.Th.); Schmp. des Hydrochlorids: 183 °C
In analoger Weise werden dargestellt:

a) 1-[3-[2-(4-Chlor-phenylsulfonylamino)ethyl]phenoxy]-2-diethylamino-ethan
aus 3-[2-[4-Chlor-phenylsulfonylamino)ethyl]phenoxyessigsäure-diethylamid.
Ausb. 77 % d.Th.; farbl. Öl, $n_D^{20}$ = 1.5552
b) 1-[3-[2-(4-Chlor-phenylsulfonylamino)ethyl]phenoxy]-2-(1-piperidyl)ethan
aus 3-[2-(4-Chlor-phenylsulfonylamino)ethyl]phenoxyessigsäure-piperidid
Ausb. 90 % d.Th.; farbl. Öl, $n_D^{20}$ = 1.5656
c) 1-[3-[2-(4-Chlor-phenylsulfonylamino)ethyl]phenoxy]-2-(1-morpholinyl)ethan
aus 3-[2-(4-Chlor-phenylsulfonylamino)ethyl]phenoxyessigsäure-morpholid
Ausb. 85 % d.Th.; farbl. Öl, $n_D^{20}$ = 1.5633
d) 1-[3-[2-(4-Chlor-phenylsulfonylamino)ethyl]phenoxy]-2-(4-methyl-1-piperazinyl)ethan
aus 3-[2-(4-Chlor-phenylsulfonylamino)ethyl]phenoxyessigsäure-(4-methylpiperazid)
Ausb. 86 % d.Th.; Schmp. des Di-maleinats: 161-163 °C. Die freie Base ist ein farbl. Öl; $n_D^{20}$ = 1.5578
e) 1-[3-[2-(4-Chlor-phenylsulfonylamino)ethyl]phenoxy]-2-(2-hydroxyethylamino)ethan
aus 3-[2-[4-Chlor-phenylsulfonylamino)ethyl]phenoxyessigsäure-(2-hydroxyethylamid)
Ausb. 73 % d.Th.; Base: farbloses Öl, $n_D^{20}$ =
f) 1-[3-[2-(4-Chlor-phenylsulfonylamino)ethyl]phenoxy]-2-[bis(2-hydroxyethyl)amino]ethan
aus 3-[2-(4-Chlor-phenylsulfonylamino)ethyl]phenoxyessigsäure-[bis-(2-hydroxyethyl)amid]
Ausb. 60 % d.Th.; farbl. Öl.

g) 1-[4-[2-(4-Chlor-phenylsulfonylamino)ethyl]phenoxy]-2-aminoethanaus 4-[2-(4-Chlor-phenylsulfonylamino)ethyl]phenoxyacetamid
Ausb. 60 % d.Th.; Schmp. Hydrochlorid: 251-253 ° C.
h) 1-[4-[2-(4-Chlor-phenylsulfonylamino)ethyl]phenoxy]-2-diethylamino-ethan
aus 4-[2-(4-Chlor-phenylsulfonylamino)ethyl]phenoxyessigsäure-diethylamid
Ausb. 58 % d.Th.; farbl. Öl.
i) 1-[4-[2-(4-Chlor-phenylsulfonylamino)ethyl]phenoxy]-2-(1-piperidyl)ethan
aus 4-[2-(4-Chlor-phenylsulfonylamino)ethyl]phenoxyessigsäure-piperidid
Ausb. 57 % d.Th.; Schmp. Hydrochlorid: 78-81 ° C
j) 1-[4-[2-(4-Chlor-phenylsulfonylamino)ethyl]phenoxy]-2-(1-morpholinyl)ethan
aus 4-[2-(4-Chlor-phenylsulfonylamino)ethyl]phenoxyessigsäure-morpholid
Ausb.: 53 % d.Th.; Schmp. Hydrochlorid: 177-179 ° C.
k) 1-[4-[2-(4-Chlor-phenylsulfonylamino)ethyl]phenoxy]-2-(2-hydroxyethylamino)ethan
aus 4-[2-(4-Chlor-phenylsulfonylamino)ethyl]phenoxyessigsäure-(2-hydroxyethylamid)
Ausb.: 62 % d.Th.; Schmp. Hydrochlorid: 206-208 ° C.
l) 1-[4-[2-(4-Chlor-phenylsulfonylamino)ethyl]phenoxy]-2-[bis(2-hydroxyethyl)amino]ethan
aus 4-[2-(4-Chlor-phenylsulfonylamino)ethyl]phenoxyessigsäure-[bis(2-hydroxyethyl)amid]
Ausb.: 44 % d.Th.; Hydrochlorid: glasartige Masse.
m) 1-[4-[2-(4-Chlor-phenylsulfonylamino)ethyl]phenyl]-3-amino-propan
aus 3-[4-[2-(4-Chlor-phenylsulfonylamino)ethyl]phenyl]propionamid
Ausb.: 74 % d.Th.; Schmp. 124 ° C (Essigester)
n) 1-[4-[2-(4-Chlor-phenylsulfonylamino)ethyl]phenoxy]-2-(1-piperidyl)ethan
aus 2-[4-[2-(4-Chlor-phenylsulfonyl-amino)ethyl]phenyl]-essigsäure-piperidid
Ausb.: 51 % d.Th.; Schmp. 76-78 ° C.
o) 1-[4-[2-(Phenylsulfonylamino)ethyl]phenoxy]-2-amino-ethan
aus 4-[2-(Phenylsulfonylamino)ethyl]phenoxyacetamid
Ausb. 56 % d. Th.; Schmp. Hydrochlorid 197-199 ° C.
p) 1-[4-[2-(Phenylsulfonylamino)ethyl]phenoxy]-2-(4-methylpiperazinyl)ethan
aus 4-[2-(Phenylsulfonylamino)ethyl]phenoxyessigsäure-(4-methyl-piperazid)
Ausb. 35 % d. Th.; Dihydrochlorid Schmp. 104-114 ° C.
q) 1-[4-[2-(4-Chlor-phenylsulfonylamino)ethyl]phenoxy]-2-(4-methylpiperazinyl)ethan
aus 4-[2-(4-Chlor-phenylsulfonylamino)ethyl]phenoxyessigsäure-(4-methyl-piperazid)
Ausb. 41 % d. Th.; Dihydrochlorid Schmp. 187-193 ° C.
r) 1-[4-[2-(4-Chlor-phenylsulfonylamino)ethyl]phenoxy]-3-(1-piperidyl)propan
aus 3-[4-[2-(4-Chlor-phenylsulfonylamino)ethyl]phenyl]propionsäure-piperidid
Ausb. 64 % d. Th.; Schmp. 87-88 ° C
s) 1-[4-[2-(4-Chlor-phenylsulfonylamino)ethyl]phenoxy]-4-amino-butan
aus 4-[4-[2-(4-Chlor-phenylsulfonylamino)ethyl]phenyl]butyramid
Ausb. 71 % d. Th.; Hydrogensulfat Schmp. 230-233 ° C.
t) 1-[3-[2-(4-Chlor-phenylsulfonylamino)ethyl]phenoxy]-4-amino-butan
aus 4-[3-[2-(4-Chlor-phenylsulfonylamino)ethyl]phenoxy] butyramid
Ausb. 66 % d. Th.; Hydrochlorid Schmp. 153 ° C.
u) 1-[3-[2-(4-Chlor-phenylsulfonylamino)ethyl]phenoxy]-2-(ethylamino)ethan
aus 3-[2-(4-Chlor-phenylsulfonylamino)ethyl]phenoxyessigsäure-ethylamid
Ausb. 48 % d. Th.; Hydrochlorid Schmp. 163-165 ° C.
v) 1-[3-[2-(4-Methyl-phenylsulfonylamino)ethyl]phenoxy]-2-(cyclohexylamino)ethan
aus 3-[2-(4-Methyl-phenylsulfonylamino)ethyl]phenoxyessigsäure-cyclohexylamid
Ausb. 85 % d. Th.; Hydrochlorid als viskoses Öl.
w) 1-[4-[2-(4-Chlor-phenylsulfonylamino)ethyl]phenoxy]-2-(ethylamino)ethan
aus 4-[2-(4-Chlor-phenylsulfonylamino)ethyl]phenoxyessigsäure-ethylamid
Ausb. 40 % d. Th., Hydrochlorid Schmp. 234-236 ° C.
x) N-(4-[2-(4-Chlor-phenylsulfonylamino)ethyl]benzyl)-piperidin
aus 4-[2-(4-Chlor-phenylsulfonylamino)ethyl]benzoesäurepiperidid
Ausb. 66 % d. Th., Hydrochlorid Schmp. 197-199 ° C

Beispiel 5

1-[4-[2-(4-Chlor-phenylsulfonylamino)ethyl]phenyl]-2-amino-ethan

Zu 0°C kaltem abs. Ether (250 ml) gibt man in kleinen Portionen 1.63 g (12 mmol) $AlCl_3$, danach 0.47 g(12 mmol) $LiAlH_4$. Zu der Suspension tropft man nun eine Lösung aus 4.1 g (12 mmol) 4-[2-(4-Chlor-phenylsulfonylamino)ethyl]benzylcyanid und 75 ml Ether. Man hält nun zwei Stdn. auf Rückflußtemperatur, kühlt ab und zersetzt mit Eis/$NaHCO_3$-Lösung. Man filtriert und extrahiert den $Al(OH)_3$-Schlamm mehrfach mit heißem Essigester. Die Essigester-Lösung wird getrocknet ($MgSO_4$) und eingedampft. Nach Umkristallisieren aus Essigester beträgt die Ausb. 2.3 g (55 % d.Th.), Schmp. 113-114°C.

In analoger Weise wird dargestellt:

a) 1-[3-[2-(4-Chlor-phenylsulfonylamino)ethyl]phenoxy]-2-amino-ethan
aus 1-[3-[2-(4-Chlor-phenylsulfonylamino)ethyl]phenoxy]-2-cyano-ethan, Schmp. 90-93°C, welches sich durch 5-stündiges Erhitzen eines Gemisches aus 3-[2-[4-Chlorphenylsulfonylamino)ethyl]phenoxy]-acetamid (1 mol), $P_2O_5$ (2 mol) und Toluol auf 110°C darstellen läßt.
Ausb. 63 % d. Th.; Schmp. 94°C

Beispiel 6

1-[3-[2-(4-Chlor-phenylsulfonylamino)ethyl]phenoxy]-2-acetamino-ethan

Man rührt eine Suspension aus 2.0 g (5.1 mmol) 1-[3-[2-(4-Chlor-phenylsulfonylamino)ethyl]phenoxy]-2-aminoethan-hydrochlorid (siehe Beipsiel 1), 50 ml Methylenchlorid und 1.56 g (15.3 mmol) abs. Triethylamin 15 min, gibt eine katalytische Menge 4-Dimethylaminopyridin zu und kühlt auf 0°C ab. Es wird nun eine Lösung aus 0.4 g (5.1 mmol) Acetylchlorid und 4 ml abs. Methylenchlorid zugetropft. Nach zwei Stunden extrahiert man zweimal mit verd. HCl und dann mit Wasser, trocknet mit $MgSO_4$ und dampft ein. Das zurückbleibende Öl wird an Kieselgel mit dem Laufmittel Methylenchlorid chromatografiert. Nach dem Eindampfen erhält man ein farbl. Öl, Ausb. 1.3 g (64 % d.Th.).

In analoger Weise erhält man mit Benzoylchlorid

a) 1-[3-[2-(4-Chlor-phenylsulfonylamino)ethyl]phenoxy]-2-benzoylamino-ethan
Ausb. 76 % d.Th., farbl. Öl.

Beispiel 7

1-[3-[2-(4-Chlorphenylsulfonylamino)ethyl]-phenoxy]-2-(cyclohexylamino)ethan

Bei Eisbadtemperatur versetzt man 5,5 g (12,2 mmol) 3-[2-(4-Chlorphenylsulfonylamino)ethyl]-phenoxyessigsäure-cyclohexylamid mit 13 ml (0,142 mol) $POCl_3$, dann entfernt man das Eisbad und rührt 20 Minuten bei Raumtemperatur. Nun wird überschüssiges $POCl_3$ bei 20° C i. Vak. abdestilliert und der Rückstand in 40 ml Ethylenglykol-dimethylether gelöst. Man kühlt wiederum auf Eisbadtemperatur ab und gibt unter starkem Rühren portionsweise 1,52 g (40 mmol) $NaBH_4$ zu. Anschließend wird 16 Stunden bei Raumtemperatur gerührt. Nach erneutem Abkühlen auf 0° C versetzt man mit 25 ml Methanol, welches 1 mol HCl im Liter enthält, und rührt eine weitere Stunde im Eisbad. Anschließend wird bei 30° C eingedampft, man gibt 50 ml 2N-$Na_2CO_3$-Lösung zu, extrahiert mit Essigester, trocknet den Extrakt mit $Na_2SO_4$ und dampft ein. Nach Zugabe von Ether und etwas Aceton wird abgesaugt und aus Ethanol umkristallisiert.
Ausbeute: 4,7 g (82 % d. Th.) Hydrochlorid, Schmp. 150-151° C.

In analoger Weise werden aus den entsprechenden Carbonamiden dargestellt:

a) 1-[3-[2-(4-Chlor-phenylsulfonylamino)ethyl]phenoxy]-3-anilino-ethan
Ausb. 66 % Hydrochlorid mit dem Schmp. 124-126°C.

b) 1-[4-[2-(4-Chlor-phenylsulfonylamino)ethyl]phenoxy]-2-(ethylamino)-ethan
Ausb. 62 % d. Th.; Schmp. 212°C.

c) 1-[3-[2-(3-Trifluormethyl-phenylsulfonylamino)ethyl]phenoxy]-2-amino-ethan
Ausb. 53 % d. Th. Hydrochlorid mit dem Schmp. 159-161°C.

d) 1-[3-[2-(4-Brom-phenylsulfonylamino)ethyl]phenoxy]-2-amino-ethan
Ausb. 48 % d. Th. Hydrochlorid mit dem Schmp. 183-185°C.

e) 1-[4-[2-(4-Trifluormethyl-phenylsulfonylamino)ethyl]phenoxy]-2-(N-alaninyl)ethan
aus 4-[2-(4-Trifluormethyl-phenylsulfonylamino)ethyl]phenoxyessigsäure-(alaninethylester)amid (Schmp.

109-114 °C) und nachfolgende Hydrolyse des Ethylesters
Ausb. 31 % d. Th.; Hydrochlorid Schmp. 187-191 °C
f) 1-[4-[2-(4-Chlor-phenylsulfonylamino)ethyl]phenoxy]-2-(N-alaninyl)ethan
aus 4-[2-(4-Chlor-phenylsulfonylamino)ethyl]phenoxyessigsäure-(alaninethylester)amid und nachfolgende Hydrolyse des Ethylesters
Ausb. 43 % d. Th.; Hydrochlorid Schmp. 117-153 °C (Zers.)
g) 1-[3-[2-(4-Chlor-phenylsulfonylaminoethyl]phenoxy]-2-(L-N-alaninyl)ethan
aus 3-[2-(4-Chlor-phenylsulfonylamino)ethyl]phenoxy]essigsäure-(L-alaninethylester)amid und nachfolgende Hydrolyse des Ethylesters
Ausb. 54 % d. Th.; Schmp. 70 °C
h) 1-[4-[2-(4-Chlor-phenylsulfonylamino)ethyl]phenyl]-2-(L-2-hydroxy-1-methyl)ethan
Ausb. 39 % d. Th.; Hydrochlorid Schmp. 181-184 °C (Ethanol).
i) 1-[3-[2-(4-Methyl-phenylsulfonylamino)ethyl]phenoxy]-2-(L-2-hydroxy-1-methyl)ethan
Ausb. 61 % d. Th.; Hydrochlorid: Hochviskoses Öl.
j) 1-[3-[2-(4-Chlor-phenylsulfonylamino)ethyl]phenoxy]-2-(L-2-hydroxy-1-phenylmethyl)ethan
Ausb. 67 % d. Th.; Hydrochlorid: Hochviskoses Öl.

Beispiel 8

1-[3-[2-(4-Chlor-phenylsulfonylamino)ethyl]phenoxy]-2-aminoethan

Zu einer Lösung aus 21,6 g (58,6 mmol) 3-[2-(4-Chlor-phenylsulfonylamino)ethyl]phenoxyacetamid und 175 ml Dimethoxyethan gibt man bei 15-20 °C portionsweise 31,3 g (0,234 mol) wasserfreies Aluminiumchlorid und läßt noch 30 min rühren. Dann gibt man bei 15-20 °C innerhalb 1/2 Std. portionsweise 8,85 g (0,234 mol) $NaBH_4$ zu. Nach drei Std. Rühren unter Kühlen werden vorsichtig 130 ml Eiswasser zugetropft, wobei die Innentemperatur 25 °C nicht überschreiten sollte. Man bringt unter Kühlen mit Eis durch Zugabe von conc. NaOH auf pH 8,5, setzt 75 ml Essigester zu und filtriert. Der Niederschlag wird mit Essigester nachgewaschen. Nach Trennung der Phasen wird die wäßrige Phase ebenfalls mit Essigester extrahiert. Die organischen Extrakte werden getrocknet, auf ca. 80 ml eingeengt und mit 20 ml HCl-haltigem Ether versetzt. Nach Kühlen saugt man ab und wäscht mit Essigester nach.
Ausbeute 16,7 g (73 % d. Th.) Hydrochlorid mit dem Schmp. 187-190 °C.

Beispiel 9

1-[3-[2-(4-Chlor-phenylsulfonylamino)ethyl]phenoxy]-3-aminopropan

1. 1-[3-[2-(Benzyloxycarbonylamino)ethyl]phenoxy]-3-(N-phthalimido)-propan
Ein Gemisch aus 24,4 g (90 mmol) 3-[2-(Benzyloxycarbonylamino)ethyl]phenol, 31,3 (117 mmol) 1-Brom-3-(N-phthalimido)propan, 16,1 g (117 mmol) pulv. trocknem Kaliumcarbonat und 100 ml abs. DMF wird 20 Std. bei 80 °C gerührt. Dann kühlt man ab, versetzt mit Eiswasser und extrahiert mehrmals mit Essigester. Die organische Phase wird nach Trocknen ($Na_2SO_4$) eingedampft und der ölige Rückstand mit einem Methanol-Wasser-Gemisch (70:30 Vol.) verrührt, wobei er kristallisierte. Nach Absaugen, Waschen mit Wasser, Lösen in Essigester und Trocknen ($Na_2SO_4$) wird erneut eingedampft, dann wird mit Isohexan verrührt, abgesaugt und getrocknet. Ausb. 36,4 g (88 % d. Th.); Schmp. 84-85 °C.
2. 1-[3-(2-Aminoethyl)phenoxy]-3-(N-phthalimido)propan
Man hydriert ein Gemisch aus 15,0 g (32,5 mmol) der nach 1. erhaltenen Verbindung, 1 l Methanol und 6,5 g Palladiumkohle (10-prozentig) 24 Std. bei Raumtemperatur und Normaldruck und saugt anschließend vom Katalysator ab. Das Filtrat wird mit HCl-haltigem Ether versetzt und eingedampft. Nach Verrühren mit Isohexan, Absaugen und Trocknen Ausbeute 9,15 g (78 % d. Th.) Hydrochlorid; Schmp. 134-136 °C (Ethanol).
3. 1-[3-[2-(4-Chlor-phenylsulfonylamino)ethyl]phenoxy]-3-(N-phthalimido)propan
Zu einer Suspension aus 6,0 g (16,6 mmol) 1-[3-(2-aminoethyl)phenoxy]-3-(N-phthalimido)propan-hydrochlorid, 70 ml Methylenchlorid und 5,06 g Trietyhlamin tropft man nach 15 min. Rühren und nach Abkühlen auf 0 °C langsam ein Gemisch aus 3,6 g (17,1 mmol) 4-Chlor-benzolsulfochlorid und 40 ml Methylenchlorid. Dann wird eine Std. bei 0 °C und eine Std. bei Raumtemperatur nachgerührt. Man verdünnt mit 100 ml Methylenchlorid und extrahiert zweimal mit verd. HCl, dann zweimal mit Wasser, trocknet ($MgSO_4$) und dampft ein. Der Rückstand wird aus Ethanol umkristallisiert. Ausb. 6,79 g (82 % d. Th.), Schmp. 118 °C.

4. Titelverbindung

Ein Gemisch aus 3,6 g (7,2 mmol) der nach 3. erhaltenen Phthalimidoverbindung, 40 ml Ethanol und 5 ml 100prozentigem Hydrazinhydrat (=100 mmol) wird 5 Std. bei 50 °C gerührt, dann wird mit sehr verd. Salzsäure auf pH 6,8 gebracht und durch $NaHCO_3$-Zugabe genau auf pH 7 eingestellt. Die Lösung wird mit Essigester extrahiert, der Extrakt mit $Na_2SO_4$ getrocknet und anschließend mit HCl-enthaltendem Ether versetzt. Man dampft ein, verrührt mit Ether, saugt ab und trocknet. Ausb. 2,3 g (79 % d. Th.) Hydrochlorid. Schmp. 174-176 °C (Wasser).

In analoger Weise werden dargestellt:

a) 1-[3-[2-(4-Brom-phenylsulfonylamino)ethyl]phenoxy]-3-amino-propan

über die Stufe

1. 1-[3-[2-(4-Brom-phenylsulfonylamino)ethyl]phenoxy]-3-(N-phthalimido)propan

(aus 4-Brom-benzolsulfochlorid und 1-[3-(2-Aminoethyl)phenoxy]-3-(N-phthalimido)propan).

Ausb. 84 % d. Th.; Schmp. 105-107 °C (Ethanol).

2. Titelverbindung

Ausb. 60 % d. Th. Hydrochlorid mit dem Schmp. 174-175 °C (Wasser)

b) 1-[3-[2-(3-Trifluormethyl-phenylsulfonylamino)ethyl]phenoxy]-3-amino-propan

Ausb. 68 % d. Th. Hydrochlorid mit dem Schmp. 151-153 °C (Wasser)

über die Stufe

1-[3-[2-(3-Trifluormethyl-phenylsulfonylamino)ethyl]phenoxy]-3-(N-phthalimido)propan

(aus 3-Trifluormethyl-benzylsulfochlorid und 1-[3-(2-Aminoethyl)phenoxy]-3-(Nphthalimido)-propan.

Ausb. 82 % d. Th.; farbloses Öl.

## Beispiel 10

1-[3-[2-(4-Methylphenylsulfonylamino)ethyl]phenoxy]-2-aminoethan

1. 3-[2-(4-Methyl-phenylsulfonylamino)ethyl]phenoxyessigsäure-benzylamid

Zu einem 40 °C warmen Gemisch aus 10,0 g (28,6 mmol) 3-[2-(4-Methyl-phenylsulfonylamino)ethyl]-phenoxyessigsäure und 100 ml abs. THF gibt man 4,64 g (28,6 mmol) Carbonylbisimidazol und läßt 10 min reagieren. Dann werden 0,4 g (28,6 mmol)p-Nitrophenol zugesetzt. Nach 10 min. fügt man 3,1 g (28,6 mmol) Benzylamin zu und rührt zwei Std. bei 60 °C. Anschließend wird THF abdestilliert, der Rückstand mit Eis und verd. HCl versetzt und mit Methylenchlorid ausgeschüttelt. Die organische Phase extrahiert man zweimal mit $NaHCO_3$-Lösung und einmal mit Wasser. Nach dem Trocknen mit $MgSO_4$ wird eingedampft und umkristallisert (Ethanol). Ausb. 10,2 g (81 % d. Th.); Schmp. 118-120 °C.

2. 1-[3-[2-(4-Methyl-phenylsulfonylamino)ethyl]phenoxy]-2-(benzylamino)-ethan

Zu einem Gemisch aus 200 ml abs. THF und 1,65 g (40 mmol) $LiAlH_4$ tropft man eine Lösung aus 100 ml abs. THF und 9,5 g (20 mmol) des nach 1. erhaltenen Amids und läßt nun 4 Std. leicht sieden. Dann wird abgekühlt und mit Eis und $NaHCO_3$-Lösung zersetzt. Man saugt nun ab, extrahiert den Filterkuchen mehrmals mit heißem Essigester und trocknet die vereinigten organischen Phasen mit $MgSO_4$. Sie werden dann eingedampft. Den Eindampfrückstand läßt man in Essigester und gibt zu der Lösung HCl-haltigen Ether. Das ausgefallene Hydrochlorid wird abgesaugt und aus Ethanol umkristallisert. Ausb. 3,9 g (39 % d. Th.) Hydrochlorid, Schmp. 154-156 °C.

3. Titelverbindung

Man hydriert ein Gemisch aus 3,5 g (7,6 mmol) der nach 2. erhaltenen Benzylaminoverbindung, 50 ml Methanol und 0,3 g 10prozentiger Palladiumkohle 48 Std. bei Raumtemperatur und Normaldruck, saugt anschließend ab, setzt zum Filtrat etwas HCl-haltigen Ether und dampft ein. Nach Verrühren mit Ether und Trocknen 2,28 g (81 % d. TH.) Hydrochlorid; Schmp. 128-130 °C.

## Beispiel 11

N-[2-[3-[2-(4-Chlor-phenylsulfonylamino)ethyl]phenoxy]ethyl]glycin

1. N-[2-[3-[2-(4-Chlor-phenylsulfonylamino)ethyl]phenoxy]ethyl]glycin-ethylester

Man rührt eine Suspension aus 5,0 g (12,8 mmol) 1-[3-[2-(4-Chlor-phenylsulfonylamino)ethyl]phenoxy]-2-aminoethanhydrochlorid, 100 ml Methylenchlorid und 3,88 g (38,3 mmol) Triethylamin 30 min. lang gibt 4,70 (28 mmol) Bromessigsäure-ethylester zu und läßt sechs Std. bei Raumtemperatur rühren. Dann wird Sodalösung zugegeben und die organische Phase abgetrennt. Man trocknet sie mit $MgSO_4$ und dampft ein. Nach Trennung mittels präparativer HPLC (Europrep RP 18, Methanol 65/Puffer 7,8 35 Vol.)

erhält man 2,5 g (41 % d. Th.) farbloses Öl.
2. Titelverbindung
Ein Gemisch aus 2,5 g (5,24 mmol) des nach 1. erhaltenen Esters in Form des Hydrochlorides, 10 ml 2N-NaOH und 10 ml Ethanol wird eine Std. bei 50 °C gerührt. Dann destilliert man das Ethanol ab, verdünnt den Rückstand mit Wasser und schüttelt zweimal mit Essigester aus. Die wäßrige Phase wird mit verd. HCl sauer gestellt, dabei fällt das kristalline Hydrochlorid aus. Nach Absaugen, Waschen mit Wasser und Trocknen Ausb. 2,2 g (94 % d. Th.) Hydrochlorid mit dem Schmp. 205-207 ° C (wäßr. Ethanol).

Beispiel 12

1-[3-[2-(4-Chlor-phenylsulfonylamino)ethyl]phenyl]-2-aminoethan

1. 1,3-Bis-(2-aminoethyl)benzol
Ein Gemisch aus 25,0 g (0,16 mmol) meta-Xylilendicyanid, 300 ml Ethanol und 50 ml conc. $NH_3$ wird in Gegenwart von Raney-Nickel 5 Std. lang bei 100 °C und 150 bar hydriert. Dann saugt man ab und dampft ein. Der Rückstand wird in Ether gelöst, die Lösung mit Aktivkohle geklärt und schließlich mit HCl-haltigem Ether versetzt, wobei das Dihydrochlorid ausfällt. Man saugt ab, wäscht mit Ether und trocknet. Ausb. 23,2, g (61 % d. Th.) Dihydrochlorid mit dem Schmp. 282-286 °C.
2. Titelverbindung
Zu einem Gemisch aus 6,0 g (25,2 mmol) des nach 1. erhaltenen Dihydrochlorides und 5,4 g (25,2 mmol) 4-Chlorbenzolsulfochlorid in 500 ml Methanol werden bei -50 °C langsam und unter heftigem Rühren 25,2 mol 2N-NaOH (50,4 mmol) zugetropft. Dann rührt man weitere 30 min, wobei man auf Raumtemperatur kommen läßt, versetzt mit Sodalösung und schüttelt mit Essigester aus. Die Essigesterphase wird getrocknet ($Na_2SO_4$) und eingedampft. Zur Reinigung wird über eine Mitteldruck-Chromatographiesäule getrennt: RP 18, Methanol: Puffer pH 7,8 = 7,3 Vol. Ausb. 3,4 g (40 % d. Th.); Schmp. 194-195 °C (Methanol).
Als Nebenprodukt fällt etwas 1,3-Bis-[2-(4-Chlor-phenylsulfonylamino)ethyl]benzol mit dem Schmp. 135-136 °C an.

Versuchsbericht

1. TX-antagonistische Wirkung auf den mit U 46619 vorkontrahierten isolierten Rattenaortenring
Methode
Die isolierte, von dem umgebenen Bindegewebe befreite Rattenaorta wird in gleich breite Ringe geschnittenen und in einem 10-ml Organbad mit Krebs-Hanseleit-Puffer bei 37 ° C superfundiert. Nach ca. 45 min. Äquilibrierungszeit werden die Aortenringe mit 0.3 µmol/l U 46619, einem stabilen Analogon des Prostaglandin-Endoperoxides $PGH_2$ (Upjohn & Co. Kalamazoo, USA), vorkontrahiert. U 46619 wurde als selektives Thromboxan-Mimetikum charakterisiert (Coleman et al., Brit. J. Pharmacol. 68, 127P., 1980). Sobald ein stabiles Plateau erreicht ist (nach ca. 15 min.), wird die Testsubstanz in steigenden Konzentrationen, beginnend mit 10nmol/l, kumulativ direkt in das Organbad zum Aortenring gegeben, bis entweder eine Endkonzentration von 100 µmol/l erreicht ist oder die Kontraktion vollständig antagonisiert ist. Berechnet wird die jeweilige prozentuale Hemmung der Kontraktion durch die Testsubstanz. Aus den Konzentrations-Wirkungs-Kurven wird die $IC_{50}$, d. h. die halbmaximale Hemmkonzentration, ermittelt.
2. Verhinderung der U 46619-bedingten Lungenembolie
Methode
Männliche NMRI Mäuse von etwa 25 g Körpergewicht werden verwendet. Die Testsubstanz wird in 1 %iger Methylcelluloselösung suspendiert und mittels Schlundsonde an die Versuchstiere verabreicht. Der Provokationstest besteht darin, daß eine für Kontrolltiere letale Dosis (800 - 1.000 µg/kg) des Thromboxan-Mimetikums (U 46619 der Firma Upjohn) schnell in die Schwanzvene injiziert wird. Die Dauer der spezifischen antagonistischen Wirkung wird getestet, indem die Tiere mit 1 mg/kg der verschiedenen Prüfsubstanzen vorbehandelt werden und die Injektion von U 46619 nach 4 h erfolgt. Die Überlebensrate gibt an, wieviele Prozent der eingesetzten Tiere die Injektion des Thromboxan-Mimetikums überlebten.
3. Hemmung des U 46619-induzierten Bronchospasmus des anaesthesierten Meerschweinchens
Methode
Meerschweinchen werden mit Narcoren anaesthesiert und mit einem Vena jugularis-Katheter und einer Trachealkanüle versehen. Die Tiere werden mit 1 ml/100 g Körpergewicht mit Raumluft maschinell

beatmet. Die Trachealkanüle ist über einen Dreiwegehahn mit einem Statham-Druck-Element verbunden, das den durch die Kontraktion der glatten Muskulatur in der Trachea entstehenden Druck aufnimmt und über eine Meßbrücke auf einen Schreiber überträgt. Nach Erreichen eines konstanten Basaltonus erhalten die Tiere 10 µg/kg U 46619 i. v. über den Vena jugularis-Katheter appliziert. Diese Dosis induziert einen schnell ansteigenden Bronchospasmus, der nach ca. 10 Minuten wieder abklingt und sehr gut reproduzierbar ist. Nach 15 Minuten erfolgt eine zweite Applikation von 10 µg/kg U 46619, in deren Verlauf nach 5 Minuten die Prüfsubstanz i. v. in verschiedenen Dosen gegeben wird. Nach weiteren 15 Minuten wird nochmals dieselbe Dosis U 46619 injiziert. Die Areas under curve über 10 min der Applikation 1 und 3 werden planimetrisch auf einer Kontron-Videoplan-Einheit bestimmt und der Bronchospasmus nach Applikation 3 in Prozent des Bronchospasmus nach Applikation 1 berechnet. Aus den Quotienten kann die $ID_{50}$ der Prüfsubstanz ermittelt werden.

Tabelle

| Substanz (Bsp. Nr.) | Überlebensrate Maus 1 mg/kg n. 4 h in % | $IC_{50}$ der vorkontrahierten isolierten Rattenaorten (µmol/l) | $ID_{50}$ des U 46619 induzierten Bronchospasmus des Meerschweinchen (mg/kg) |
|---|---|---|---|
| Bsp. 1 r | 90 | 1.0 | 0.02 |
| Bsp. 1 g | 100 | 1.6 | 0.04 |
| Bsp. 2 a | 100 | 1.8 | 0.01 |
| Bsp. 1 d | 100 | 3.4 | 0.50 |
| Bsp. 1 f | 100 | 1.6 | 0.02 |
| Bsp. 4 | 100 | 0.34 | 0.01 |
| Bsp. 5 | 100 | 0.10 | 0.05 |
| Bsp. 4 m | 100 | 3.00 | 0.05 |
| Bsp. 4 s | 90 | 2.6 | 0.10 |

## Patentansprüche

**1.** Verbindungen der allgemeinen Formel I

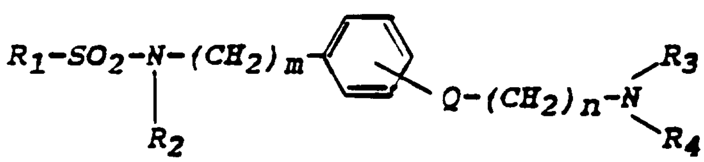

(I),

in welcher

- $R_1$ eine Aryl-, Aralkyl oder eine Aralkenylgruppe, deren Arylrest jeweils ein- oder mehrfach durch Halogen, Cyan, Alkyl, Trifluormethyl, Alkoxy, Alkylthio, Trifluoromethoxy, Hydroxy oder Carboxy substituiert sein kann,
- m eine ganze Zahl 2,
- n eine ganze Zahl von 2 bis 5,
- $R_2$ Wasserstoff,
- Q eine Bindung oder ein Sauerstoffatom,
- $R_3$ Wasserstoff oder eine $C_1$-$C_4$ Alkylgruppe, welche ggf. endständig durch Carboxyl oder durch eine Hydroxygruppe substituiert ist und
- $R_4$ Wasserstoff, eine niedere Alkylgruppe mit 1-4 C-Atomen, welche ggf. endständig durch Carboxyl oder Hydroxyl substituiert ist, eine ggf. substituierte Phenyl-, Pyridinyl-, Pyrimidinyl-, Cycloalkyl- oder Acyl-Gruppe

  oder eine Gruppe

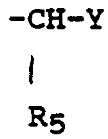

in welcher $R_5$ eine geradkettige oder verzweigte Alkylkette mit 1-4 C-Atomen darstellt, welche ggf. endständig durch Carboxyl, Alkoxycarbonyl, Aminocarbonyl, Hydroxy, Mercapto, Alkylthio oder Imidazolyl substituiert ist und

Y eine Carboxy, eine Alkoxycarbonyl, Aminocarbonyl- oder Cyano, Formyl, Hydroxymethyl, Aminomethyl oder eine Orthoestergruppe darstellt, bedeuten

wobei $R_3$ und $R_4$ auch Bestandteil eines 5- oder 6-gliedrigen gesättigten oder ungesättigten, ggf. substituierten Heterozyclus mit 1-4 Heteroatomen sein kann, der mit weiteren Ringverbindungen über eine oder mehrere Bindungen annelliert sein kann.

sowie deren physiologisch unbedenkliche Salze, Ester und Amide.

**2.** Verfahren zur Herstellung von Verbindungen der Formel I

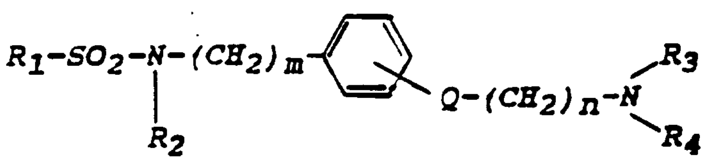

(I),

in welcher

- $R_1$ eine Aryl-, Aralkyl oder eine Aralkenylgruppe, deren Arylrest jeweils ein- oder mehrfach durch Halogen, Cyan, Alkyl, Trifluormethyl, Alkoxy, Alkylthio, Trifluoromethoxy, Hydroxy oder Carboxy substituiert sein kann,
- m eine ganze Zahl 2,
- n eine ganze Zahl von 2 bis 5,
- $R_2$ Wasserstoff,
- Q eine Bindung oder ein Sauerstoffatom,
- $R_3$ Wasserstoff oder eine $C_1$-$C_4$ Alkylgruppe, welche ggf. endständig durch Carboxyl oder durch eine Hydroxygruppe substituiert ist und
- $R_4$ Wasserstoff, eine niedere Alkylgruppe mit 1-4 C-Atomen, welche ggf. endständig durch Carboxyl oder Hydroxyl substituiert ist, eine ggf. substituierte Phenyl-, Pyridinyl-, Pyrimidinyl-, Cycloalkyl- oder Acyl-Gruppe oder eine Gruppe

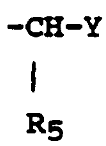

in welcher $R_5$ eine geradkettige oder verzweigte Alkylkette mit 1-4 C-Atomen darstellt, welche ggf. endständig durch Carboxyl, Alkoxycarbonyl, Aminocarbonyl, Hydroxy, Mercapto, Alkylthio oder Imidazolyl substituiert ist und

Y eine Carboxy, eine Alkoxycarbonyl, Aminocarbonyl- oder Cyano, Formyl, Hydroxymethyl, Aminomethyl oder eine Orthoestergruppe darstellt, bedeuten

wobei $R_3$ und $R_4$ auch Bestandteil eines 5- oder 6-gliedrigen gesättigten oder ungesättigten, ggf. substituierten Heterozyclus mit 1-4 Heteroatomen sein kann, der mit weiteren Ringverbindungen über eine oder mehrere Bindungen annelliert sein kann,

sowie deren physiologisch unbedenkliche Salze, Ester und Amide, dadurch gekennzeichnet, daß man in an sich bekannter Weise

a) eine Verbindung der Formel II

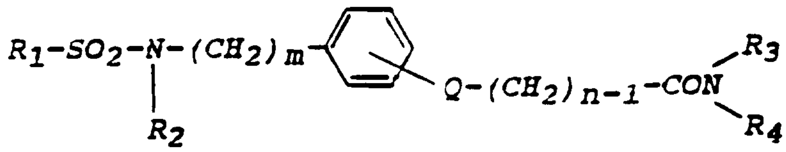

(II),

in der $R_1$, $R_2$, $R_3$, $R_4$, Q, m und n die oben angegebenen Bedeutungen haben, zum Amin reduziert, oder

b) Verbindungen der nachstehenden Formeln III-VII reduziert, in denen $R_1$, $R_2$, m, n und Q die angegebenen Bedeutungen haben,

1. Azide der Formel III

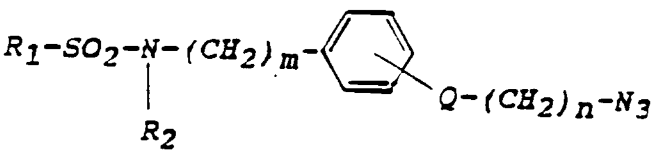

(III),

2. Nitroalkane der Formel IV

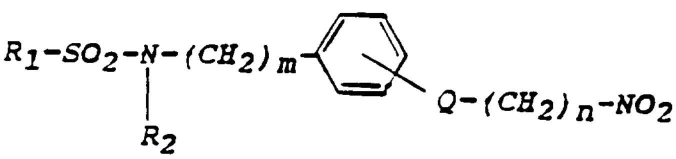

(IV),

3. Nitrile der Formel V

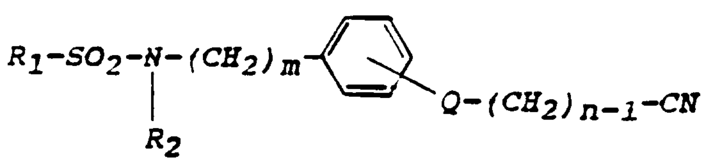

(V),

4. Oxime der Formel VI

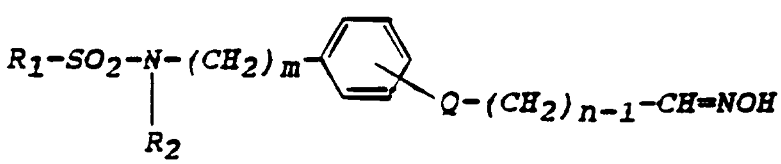

(VI),

wobei jeweils primäre Amine (I, $R_3 = R_4 = H$) entstehen, oder

5. Schiff-Basen der Formel VII

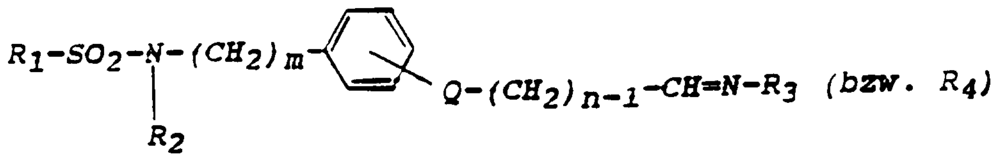

(VII),

wobei sekundäre Amine entstehen,
und anschließend gewünschtenfalls die erhaltenen Amine der Formel I in andere Verbindungen der Formel I überführt, sowie gegebenenfalls die erhaltenen Verbindungen in physiologisch unbedenkliche Salze, Ester und Amide überführt.

**3.** Arzneimittel, enthaltend mindestens eine Verbindung gemäß Anspruch 1 neben üblichen Träger- und Hilfsstoffen.

**4.** Verwendung von Verbindungen gemäß Anspruch 1 zur Herstellung von Arzneimitteln mit Thromboxan-antagonistischer Wirkung.

**5.** Verbindungen der Formel II

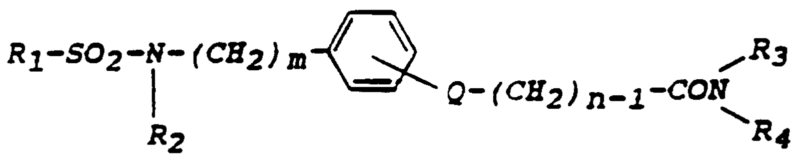

(II),

in welcher
- $R_1$ eine 4-Chlorphenyl-Gruppe,
- m die Zahl 2,
- n eine ganze Zahl von 2 bis 5,
- R Wasserstoff,
- Q eine Bindung oder ein Sauerstoffatom,
- $R_3$ ein Wasserstoffatom oder eine $C_1$-$C_4$-Alkylgruppe, welche gegebenenfalls endständig durch eine Hydroxygruppe substituiert ist,
- $R_4$ Wasserstoff, eine niedere Alkylgruppe mit 1 - 4 C-Atomen, welche gegebenenfalls endständig durch Hydroxyl substituiert ist, oder eine gegebenenfalls substituierte Phenyl-, Pyridinyl-, Pyrimidinyl-, Cycloalkyl- oder Acylgruppe bedeuten,

wobei $R_3$ und $R_4$ auch Bestandteile eines 5- oder 6-gliedrigen gesättigten oder ungesättigten, gegebenenfalls substituierten Heterozyclus mit 1 - 4 Heteroatomen sein kann, der mit weiteren Ringverbindungen über eine odere mehrere Bindungen annelliert sein kann.

**6.** Verbindungen gemäß Anspruch 6, ausgewählt aus der Gruppe:
3-[4-[2-(4-Chlorphenylsulfonylamino)ethyl]phenyl]-propionamid
4-[2-(4-Chlorphenylsulfonylamino)ethyl]phenylessigsäureamid
4-[2-(4-Chlorphenylsulfonylamino)ethyl-phenylessigsäure-ethylamid
3-[2-(4-Chlorphenylsulfonylamino)ethyl]phenoxyacetylpiperidid
3-[2-(4-Chlorphenylsulfonylamino)ethyl]phenoxyacetyl-diethylamid
3-[2-(4-Chlorphenylsulfonylamino)ethyl]phenoxyessigsäure-(2-hydroxyethylamid)
3-[2-(4-Chlorphenylsulfonylamini)ethyl]phenoxyessigsäure]-2-[bis(2-hydroxyethyl)amid]
3-[2-(4-Chlorphenylsulfonylamino)ethyl]phenoxyessigsäure-morpholid

3-[2-(4-Chlorphenylsulfonylamino)ethyl]phenoxyessigsäure-(4-methyl-piperazid)
3-[2-(4-Chlorphenylsulfonylamino)ethyl]phenoxyessigsäure-anilid
3-[2-(4-Chlorphenylsulfonylamino)ethyl]phenoxyessigsäure-cyclohexylamid
4-[2-(4-Chlorphenylsulfonylamino)ethyl]phenoxyessigsäure-diethylamid
4-[2-(4-Chlorphenylsulfonylamino)ethyl]phenoxyessigsäure-ethylamid
4-[2-(4-Chlorphenylsulfonylamino)ethyl]phenoxyessigsäure-(4-methyl-piperazid)
4-[2-(4-Chlorphenylsulfonylamino)ethyl]phenoxyessigsäure-(2-pyrimidinyl)amid
4-[2-(4-Chlorphenylsulfonylamino)ethyl]phenoxyessigsäure-(3-pyridyl)amid
4-[3-[2-(4-Chlorphenylsulfonylamino)ethyl]phenoxy]butyramid
3-[2-(4-Chlorphenylsulfonylamino)ethyl]phenoxyessigsäure-ethylamid
4-[2-(4-Chlorphenylsulfonylamino)ethyl]phenylessigsäure-(4-pyridyl)amid
3-[4-[2-(4-Chlorphenylsulfonylamino)ethyl]phenyl]propionsäure-piperidid
4-[2-[4-Chlorphenylsulfonylamino)ethyl]phenoxyessigsäure-[bis(2-hydroxyethyl)amid]
4-[2-(4-Chlorphenylsulfonylamino)ethyl]phenoxyessigsäure-(2-hydroxyethylamid)
4-[2-(4-Chlor-phenylsulfonylamino)ethyl]phenoxyessigsäure-piperidid
4-[2-(4-Chlorphenylsulfonylamino)ethyl]phenoxyessigsäure-morpholid
4-[2-(4-Chlorphenylsulfonylamino)ethyl]phenoxyessigsäureamid

## Claims

**1.** Compounds of the general formula I

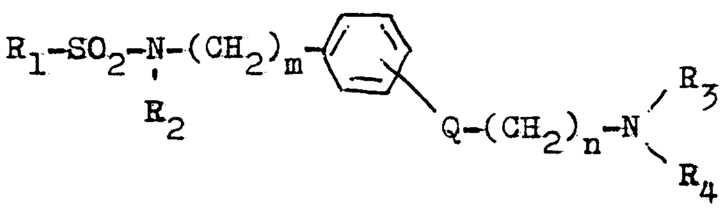

(I)

in which $R_1$ signifies an aryl, aralkyl or an aralkenyl group, the aryl radical of which can, in each case, be substituted one or more times by halogen, cyano, alkyl, trifluoromethyl, alkoxy, alkylthio, trifluoromethoxy, hydroxyl or carboxyl, m a whole number 2, n a whole number from 2 to 5, $R_2$ hydrogen, Q a bond or an oxygen atom, $R_3$ hydrogen or a $C_1$-$C_4$ alkyl group which is possibly terminally substituted by a carboxyl or by a hydroxyl group and $R_4$ hydrogen, a lower alkyl group with 1 - 4 C-atoms, which is possibly terminally substituted by carboxyl or hydroxyl, a possibly substituted phenyl, pyridinyl, pyrimidinyl, cycloalkyl or acyl group or a group

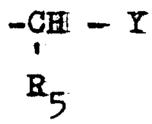

in which $R_5$ represents a straight-chained or branched alkyl chain with 1 - 4 C-atoms which is possibly terminally substituted by carboxyl, alkoxycarbonyl, hydroxyl, mercapto, alkylthio or imidazolyl and Y represents a carboxyl, an alkoxycarbonyl, aminocarbonyl or cyano, formyl, hydroxymethyl, aminomethyl or an ortho ester group, whereby $R_3$ and $R_4$ can also be components of a 5- or 6-membered saturated or unsaturated possibly substituted heterocycle with 1 - 4 heteroatoms which can be annellated with further ring compounds via one or more bonds, as well as their physiologically acceptable salts, esters and amides.

**2.** Process for the preparation of compounds of the formula I

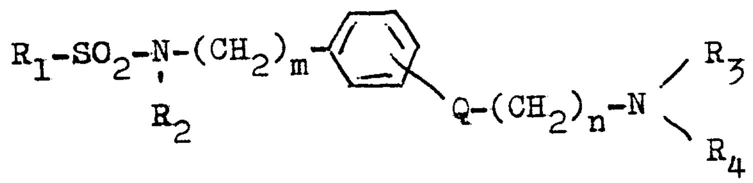

(I)

in which $R_1$ signifies an aryl, aralkyl or an aralkenyl group, the aryl radical of which can, in each case, be substituted one or more times by halogen, cyano, alkyl, trifluoromethyl, alkoxy, alkylthio, trifluoromethoxy, hydroxyl or carboxyl, m a whole number 2, n a whole number from 2 to 5, $R_2$ hydrogen, Q a valency bond or an oxygen atom, $R_3$ hydrogen or a $C_1$-$C_4$-alkyl group which is possibly terminally substituted by carboxyl or by a hydroxyl group and $R_4$ hydrogen, a lower alkyl group with 1 - 4 C-atoms, which is possibly substituted terminally by carboxyl or hydroxyl, a possibly substituted phenyl, pyridinyl, pyrimidinyl, cycloalkyl or acyl group or a group

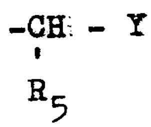

in which $R_5$ represents a straight-chained or branched alkyl chain with 1 - 4 C-atoms which is possibly terminally substituted by carboxyl, alkoxycarbonyl, aminocarbonyl, hydroxyl, mercapto, alkylthio or imidazolyl and Y a carboxyl, an alkoxycarbonyl, aminocarbonyl or cyano, formyl, hydroxymethyl, aminomethyl or an ortho ester group, whereby $R_3$ and $R_4$ can also be components of a 5- or 6-membered heterocycle with 1 - 4 heteroatoms which can be annellated with further ring compounds via one or more bonds, as well as of their physiologically acceptable salts, esters and amides, characterised in that, in per se known manner, one

a) reduces a compound of the formula II

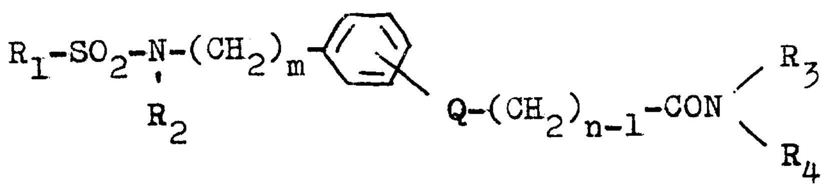

(II)

in which $R_1$, $R_2$, $R_3$, $R_4$, Q, m and n have the above-given meanings, to the amine or

b) reduces compounds of the following formulae III - VII, in which $R_1$, $R_2$, m, n and Q have the above-given meanings,

1. azides of the formula III

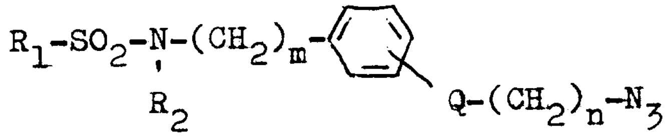

(III)

2. nitroalkanes of the formula IV

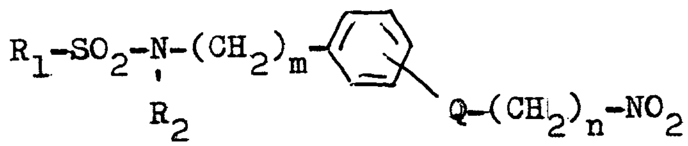

(IV)

3. nitriles of the formula V

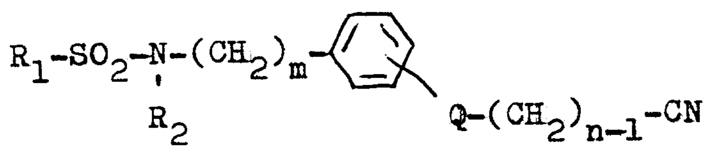

(V)

4. oximes of the formula VI

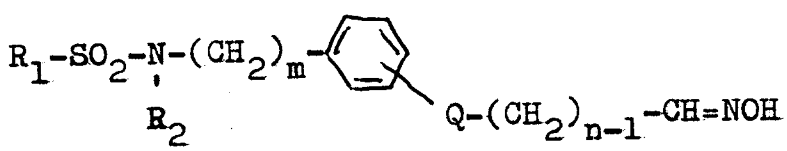

(VI)

whereby, in each case, primary amines (I, $R_3 = R_4 = H$) result or
5. Schiff's bases of the formula VII

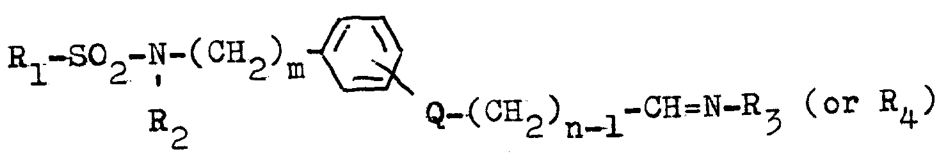

(VII)

whereby secondary amines result,
and subsequently, if desired, converts the amines obtained of the formula I into other compounds of the formula I, as well as possibly converts the compounds obtained into physiologically acceptable salts, esters and amides.

**3.** Medicaments containing at least one compound according to claim 1, besides usual carrier and adjuvant materials.

**4.** Use of compounds according to claim 1 for the preparation of medicaments with thromboxane-antagonistic action.

**5.** Compounds of the formula II

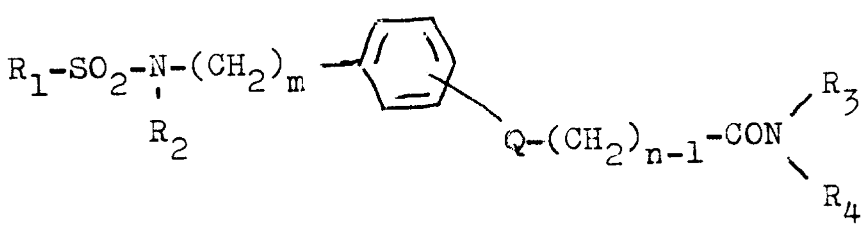

(II)

in which $R_1$ signifies a 4-chlorophenyl group, m the number 2, n a whole number from 2 to 5, R hydrogen, Q a bond or an oxygen atom, $R_3$ a hydrogen atom or a $C_1$-$C_4$-alkyl group which is possibly terminally substituted by a hydroxyl group, $R_4$ hydrogen, a lower alkyl group with 1 - 4 C-atoms which is possibly terminally substituted by hydroxyl or a possibly substituted phenyl, pyridinyl, pyrimidinyl, cycloalkyl or acyl group, whereby $R_3$ and $R_4$ can also be components of a 5- or 6-membered saturated or unsaturated, possibly substituted heterocycle with 1 - 4 heteroatoms which can be annellated with further ring compounds via one or more bonds.

**6.** Compounds according to claim 6 selected from the group:
3-[4-[2-(4-chlorophenylsulphonylamino)-ethyl]-phenyl]-propionamide
4-[2-(4-chlorophenylsulphonylamino)-ethyl]-phenylacetic acid amide
4-[2-(4-chlorophenylsulphonylamino)-ethyl]-phenylacetic acid ethylamide
3-[2-(4-chlorophenylsulphonylamino)-ethyl]-phenoxyacetyl piperidide
3-[2-(4-chlorophenylsulphonylamino)-ethyl]-phenoxyacetyl diethylamide
3-[2-(4-chlorophenylsulphonylamino)-ethyl]-phenoxyacetic acid (2-hydroxyethylamide)
3-[2-(4-chlorophenylsulphonylamino)-ethyl]-phenoxyacetic acid] 2-[bis-(2-hydroxyethyl)-amide]
3-[2-(4-chlorophenylsulphonylamino)-ethyl]-phenoxyacetic acid morpholide
3-[2-(4-chlorophenylsulphonylamino)-ethyl]-phenoxyacetic acid (4-methylpiperazide)
3-[2-(4-chlorophenylsulphonylamino)-ethyl]-phenoxyacetic acid anilide
3-[2-(4-chlorophenylsulphonylamino)-ethyl]-phenoxyacetic acid cyclohexylamide
4-[2-(4-chlorophenylsulphonylamino)-ethyl]-phenoxyacetic acid diethylamide
4-[2-(4-chlorophenylsulphonylamino)-ethyl]-phenoxyacetic acid ethylamide
4-[2-(4-chlorophenylsulphonylamino)-ethyl]-phenoxyacetic acid (4-methylpiperazide)
4-[2-(4-chlorophenylsulphonylamino)-ethyl]-phenoxyacetic acid (2-pyrimidinyl)-amide
4-[2-(4-chlorophenylsulphonylamino)-ethyl]-phenoxyacetic acid (3-pyridyl)-amide
4-[3-[2-(4-chlorophenylsulphonylamino)-ethyl]-phenoxy]-butyramide
3-[2-(4-chlorophenylsulphonylamino)-ethyl]-phenoxyacetic acid ethylamide
4-[2-(4-chlorophenylsulphonylamino)-ethyl]-phenylacetic acid (4-pyridyl)-amide
3-[4-[2-(4-chlorophenylsulphonylamino)-ethyl]-phenyl]-propionic acid piperidide
4-[2-[4-chlorophenylsulphonylamino)-ethyl]-phenoxyacetic acid [bis-(2-hydroxyethyl)-amide]
4-[2-(4-chlorophenylsulphonylamino)-ethyl]-phenoxyacetic acid (2-hydroxyethylamide)
4-[2-(4-chlorophenylsulphonylamino)-ethyl]-phenoxyacetic acid piperidide
4-[2-(4-chlorophenylsulphonylamino)-ethyl]-phenoxyacetic acid morpholide
4-[2-(4-chlorophenylsulphonylamino)-ethyl]-phenoxyacetic acid amide

## Revendications

**1.** Composés de formule générale I

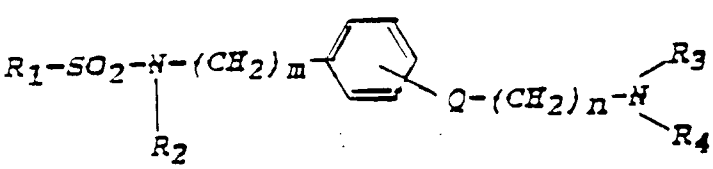

(I),

dans laquelle

- $R_1$ représente un groupe aryle, aralkyle ou aralcényle, dont le reste aryle peut être substitué une ou plusieurs fois par un groupe halogéno, cyano, alkyle, trifluorométhyle, alcoxy, alkylthio, trifluorométhoxy, hydroxy ou carboxy,
- m vaut un nombre entier de 2,
- n vaut un nombre entier de 2 à 5,
- $R_2$ représente un atome d'hydrogène,
- Q représente une valence libre ou un atome d'oxygène,
- $R_3$ représente un atome d'hydrogène ou un groupe alkyle en $C_1$-$C_4$, qui peut être éventuellement substitué en bout de chaîne par un groupe carboxyle ou hydroxyle, et

$R_4$ représente un atome d'hydrogène, un groupe alkyle inférieur en $C_1$-$C_4$, qui peut être éventuellement substitué en bout de chaîne par un groupe carboxyle ou hydroxyle, un groupe phényle, pyridinyle, pyrimidinyle, cycloalkyle ou acyle éventuellement substitué,

ou un groupe

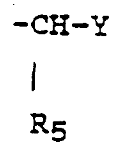

dans lequel $R_5$ représente une chaîne alkylique linéaire ou ramifiée, ayant 1-4 atomes de carbone, qui peut être éventuellement substituée en bout de chaîne par un groupe carboxyle, alcoxycarbonyle, aminocarbonyle, hydroxy, mercapto, alkylthio ou imidazolyle, et

Y représente un groupe carboxy, alcoxycarbonyle, aminocarbonyle ou cyano, formyle, hydroxyméthyle, aminométhyle ou orthoester,

$R_3$ et $R_4$ pouvant également faire partie d'un hétérocycle à 5 ou 6 chaînons, saturé ou insaturé, éventuellement substitué, comportant 1-4 hétéroatomes, qui peut être condensé à d'autres composés cycliques par l'intermédiaire d'une ou plusieurs liaisons,

ainsi que leurs sels, esters et amides physiologiquement acceptables.

2. Procédé de préparation de composés de formule générale I

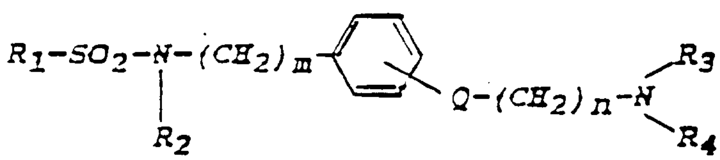

(I),

dans laquelle

- $R_1$ représente un groupe aryle, aralkyle ou aralcényle, dont le reste aryle peut être substitué une ou plusieurs fois par un groupe halogéno, cyano, alkyle, trifluorométhyle, alcoxy, alkylthio, trifluorométhoxy, hydroxy ou carboxy,
- m vaut un nombre entier de 2,
- n vaut un nombre entier de 2 à 5,
- $R_2$ représente un atome d'hydrogène,
- Q représente une valence libre ou un atome d'oxygène,
- $R_3$ représente un atome d'hydrogène ou un groupe alkyle en $C_1$-$C_4$, qui peut être éventuellement substitué en bout de chaîne par un groupe carboxyle ou hydroxyle, et
- $R_4$ représente un atome d'hydrogène, un groupe alkyle inférieur en $C_1$-$C_4$, qui peut être éventuellement substitué en bout de chaîne par un groupe carboxyle ou hydroxyle, un groupe phényle, pyridinyle, pyrimidinyle, cycloalkyle ou acyle éventuellement substitué,

ou un groupe

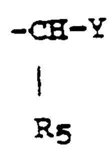

dans lequel $R_5$ représente une chaîne alkylique linéaire ou ramifiée, ayant 1-4 atomes de carbone, qui peut être éventuellement substituée en bout de chaîne par un groupe carboxyle, alcoxycarbonyle, aminocarbonyle, hydroxy, mercapto, alkylthio ou imidazolyle, et

Y représente un groupe carboxy, alcoxycarbonyle, aminocarbonyle ou cyano, formyle, hydroxy-

méthyle, aminométhyle ou orthoester,

$R_3$ et $R_4$ pouvant également faire partie d'un hétérocycle à 5 ou 6 chaînons, saturé ou insaturé, éventuellement substitué, comportant 1-4 hétéroatomes, qui peut être condensé à d'autres composés cycliques par l'intermédiaire d'une ou plusieurs liaisons,

ainsi que de leurs sels, esters et amides physiologiquement acceptables, caractérisé en ce que, de manière connue en soi,

a) on réduit un composé de formule II

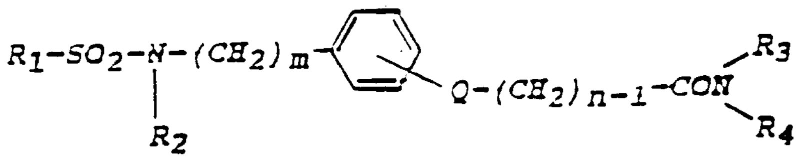

(II),

dans laquelle

$R_1$, $R_2$, $R_3$, $R_4$, Q, m et n ont les significations mentionnées ci-dessus, en amine, ou

b) on réduit des composés de formules III-VII, représentées ci-après, dans lesquelles $R_1$, $R_2$, m, n et Q ont les significations mentionnées ci-dessus,

1. des azides de formule III

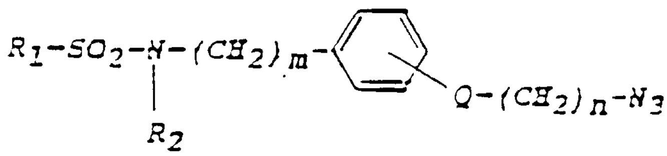

(III),

2. des nitroalcanes de formule IV

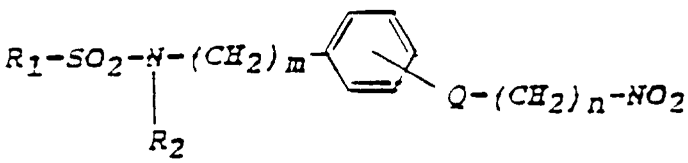

(IV),

3. des nitriles de formule V

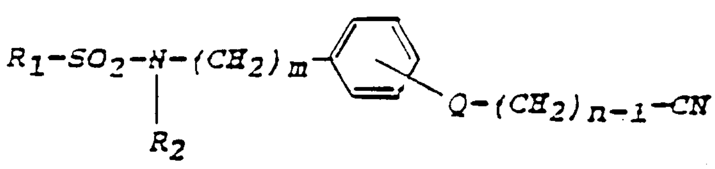

(V),

4. des oximes de formule VI

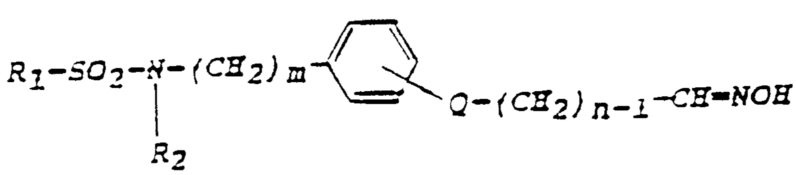

(VI),

en obtenant dans chaque cas une amine primaire (I, $R_3$ = $R_4$ = H),
ou
5. des bases de Schiff de formule VII

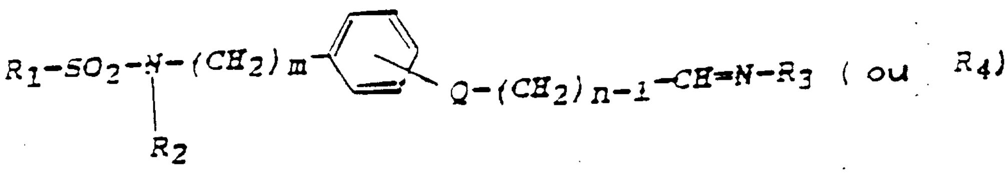

(VII),

en obtenant des amines secondaires,
et ensuite, on transforme éventuellement l'amine de formule I obtenue en d'autres composés de formule I, et on transforme éventuellement les composés obtenus en leurs sels, esters et amides physiologiquement acceptables.

**3.** Médicament, contenant au moins un composé selon la revendication 1, en plus de véhicules et d'adjuvants usuels.

**4.** Utilisation de composés selon la revendication 1, pour la préparation de médicaments ayant une activité anti-thromboxane.

**5.** Composés de formule générale II

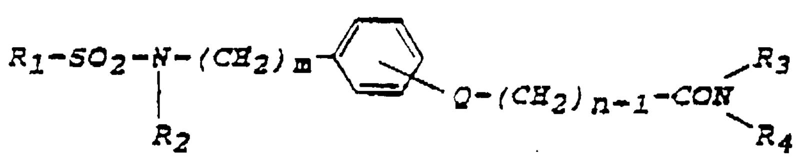

(II),

dans laquelle
- $R_1$ représente un groupe 4-chlorophényle,
- m vaut 2,
- n vaut un nombre entier de 2 à 5,
- R représente un atome d'hydrogène,
- Q représente une valence libre ou un atome d'oxygène,
- $R_3$ représente un atome d'hydrogène ou un groupe alkyle en $C_1$-$C_4$, qui peut être éventuellement substitué en bout de chaîne par un groupe hydroxyle, et
- $R_4$ représente un atome d'hydrogène, un groupe alkyle inférieur en $C_1$-$C_4$, qui peut être éventuellement substitué en bout de chaîne par un groupe hydroxyle, ou par un groupe phényle, pyridinyle, pyrimidinyle, cycloalkyle ou acyle éventuellement substitué,

$R_3$ et $R_4$ pouvant également faire partie d'un hétérocycle à 5 ou 6 chaînons, saturé ou insaturé, éventuellement substitué, comportant 1-4 hétéroatomes, qui peut être condensé à d'autres composés cycliques par l'intermédiaire d'une ou plusieurs liaisons.

**6.** Composés selon la revendication 6, choisi dans le groupe :
3-[4-[2-(4-chlorophénylsulfonylamino)éthyl]phényl]-propionamide
amide de l'acide 4-[2-(4-chlorophénylsulfonylamino)éthyl]phénylacétique
éthylamide de l'acide 4-[2-(4-chlorophénylsulfonylamino)éthyl]phénylacétique
3-[2-(4-chlorophénylsulfonylamino)éthyl]phénoxyacétylpipéridide
3-[2-(4-chlorophénylsulfonylamino)éthyl]phénoxyacétyldiéthylamide
(2-hydroxyéthylamide) de l'acide 3-[2-(4-chlorophénylsulfonylamino)éthyl]-phénoxyacétique
2-[bis(2-hydroxyéthyl)amide)] de l'acide 3-[2-(4-chlorophénylsulfonylamino)éthyl]-phénoxyacétique

morpholide de l'acide 3-[2-(4-chlorophénylsulfonylamino)éthyl]-phénoxyacétique
(4-méthylpipérazide) de l'acide 3-[2-(4-chlorophénylsulfonylamino)éthyl]-phénoxyacétique
anilide de l'acide 3-[2-(4-chlorophénylsulfonylamino)éthyl]-phénoxyacétique
cyclohexylamide de l'acide 3-[2-(4-chlorophénylsulfonylamino)éthyl]-phénoxyacétique
diéthylamide de l'acide 4-[2-(4-chlorophénylsulfonylamino)éthyl]-phénoxyacétique
éthylamide de l'acide 4-[2-(4-chlorophénylsulfonylamino)éthyl]-phénoxyacétique
(4-méthylpipérazide) de l'acide 4-[2-(4-chlorophénylsulfonylamino)éthyl]-phénoxyacétique
(2-pyrimidinyl)amide de l'acide 4-[2-(4-chlorophénylsulfonylamino)éthyl]-phénoxyacétique
(3-pyridyl)amide de l'acide 4-[2-(4-chlorophénylsulfonylamino)éthyl]-phénoxyacétique
4-[3-[2-(4-chlorophénylsulfonylamino)éthyl]-phénoxybutyramide
éthylamide de l'acide 3-[2-(4-chlorophénylsulfonylamino)éthyl]-phénoxyacétique
(4-pyridyl)amide de l'acide 4-[2-(4-chlorophénylsulfonylamino)éthyl]-phénylacétique
pipéridide de l'acide 3-[4-[2-(4-chlorophénylsulfonylamino)éthyl]-phényl]-propionique
[bis(2-hydroxyéthyl)amide] de l'acide 4-[2-(4-chlorophénylsulfonylamino)-éthyl]-phénoxyacétique
(2-hydroxyéthyl)amide) de l'acide 4-[2-(4-chlorophénylsulfonylamino)-éthyl]-phénoxyacétique
pipéridide de l'acide 4-[2-(4-chlorophénylsulfonylamino)-éthyl]-phénoxyacétique
morpholide de l'acide 4-[2-(4-chlorophénylsulfonylamino)-éthyl]-phénoxyacétique
4-[2-(4-chlorophénylsulfonylamino)-éthyl]-phénylacétamide